# EUROPEAN PATENT APPLICATION

(11) **EP 4 744 721 A1**
(43) Date of publication of application: **20.05.2026**
(21) Application number: 24842184.4
(22) Date of filing: 28.06.2024
(51) Int. Cl.: A61N 1/375, A61N 1/39, A61N 1/36, A61M 5/142

(54) **ANTI-REVERSE FIXING STRUCTURE AND IMPLANTABLE MEDICAL DEVICE**

(30) Priority: 14.07.2023 CN 202310870034; 14.07.2023 CN 202321862102 U
(71) Applicant: MicroPort Soaring CRM (Shanghai) Co., Ltd., Shanghai 201203 (CN)
(72) Inventor: WU, Nan, Shanghai 201203 (CN); CHENG, Zhijun, Shanghai 201203 (CN); ZHU, Xiaoming, Shanghai 201203 (CN); YU, Peng, Shanghai 201203 (CN)
(74) Representative: Lavoix
(86) International application number: PCT/CN2024/102384
(87) International publication number: WO 2025/016188

(57) **Abstract**

Provided herein are an anti-reverse fixing structure and an implantable medical device. The anti-reverse fixing structure includes: a fixation substructure for fixing an implantable medical device to target organic tissue; an anti-reverse substructure; and a mounting substructure for being connected to a distal end of the implantable medical device. The fixation substructure is connected to the mounting substructure through the anti-reverse substructure. The anti-reverse substructure includes at least one first anti-reverse feature, which when stressed at its distal end by an external force acting along an axis of the anti-reverse fixing structure from a distal end to a proximal end thereof, is deformable so as to come into abutment, at least in part, with the target organic tissue. With this anti-reverse fixing structure, higher reliability and increased safety can be obtained after the implantable medical device is implanted to the target organic tissue. Moreover, the anti-reverse fixing structure is simple in structure and easy to fabricate and assemble.

## Description

### TECHNICAL FIELD

The present application relates to the field of medical devices and, in particular, to an anti-reverse fixing structure and an implantable medical device.

### BACKGROUND

At present, most leadless pacemakers are fixed either passively by tines, or actively using a helix. The helix-based active fixation approach has found increasingly extensive use thanks to its advantages of a wide range of fixation site options, high stability and high reliability. In the helix-based active fixation approach, an anti-reverse structure may be employed to obtain increased fixation stability and reliability. Fixation elements in conventional leadless pacemakers are generally only capable of preventing unscrewing of the leadless pacemaker away from myocardial tissue, but cannot adequately prevent it from incrementally moving deeper into myocardial tissue. Such incrementally deeper movement presents a risk of ending up with perforation of the myocardial tissue, which may potentially lead to safety hazards. Moreover, the conventional fixation elements with anti-reverse capabilities are generally complex and difficult to fabricate or assemble.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

The present application seeks to solve the problem that conventional fixation elements with anti-reverse capabilities may cause tissue perforation by an implantable medical device in which the fixation element is employed and are complex in structure, by presenting an anti-reverse fixing structure and an implantable medical device. This application enables more stable fixation of an implantable medical device to target organic tissue, with a reduced risk of perforation caused by the implantable medical device.

The above object is attained by an anti-reverse fixing structure provided herein for use with an implantable medical device, which includes: a fixation substructure for fixing the implantable medical device to target tissue; an anti-reverse substructure; and a mounting substructure for being coupled to a distal end of the implantable medical device.

The fixation substructure is coupled to the mounting substructure through the anti-reverse substructure.

The anti-reverse substructure includes at least one first anti-reverse feature, which is deformable when stressed at its distal end by an external force acting along an axis of the anti-reverse fixing structure from a distal end to a proximal end thereof.

The above object is also attained by an implantable medical device provided herein, which includes a device body, an electrode, a feedthrough and the anti-reverse fixing structure as defined above.

The electrode is coupled to a distal end of the anti-reverse fixing structure, and the anti-reverse fixing structure is coupled at a proximal end thereof to a distal end of the device body. The feedthrough includes at least one feedthrough wire, which is passed through the anti-reverse fixing structure and electrically connected to the electrode.

Optionally, the implantable medical device may further include a drug pill disposed between the electrode and the anti-reverse fixing structure.

The anti-reverse fixing structure and the implantable medical device provided herein offer at least the following benefits:
The anti-reverse fixing structure includes: a fixation substructure for fixing an implantable medical device to target tissue; an anti-reverse substructure; and a mounting substructure for being coupled to a distal end of the implantable medical device. With this arrangement, not only the anti-reverse fixing structure can easily fix the implantable medical device to target organic tissue, but a modular design is provided, which makes the anti-reverse fixing structure simple in structure and easy to fabricate, assemble and maintain, resulting in manpower, material and time savings. Additionally, in the anti-reverse fixing structure, the anti-reverse substructure includes at least one first anti-reverse feature, which, when stressed at its distal end by an external force acting along an axis of the anti-reverse fixing structure from a distal end to a proximal end thereof, will bend at its distal end outwardly away from the axis of the anti-reverse fixing structure so as to come into abutment, at least in part, with the target organic tissue. With this arrangement, the anti-reverse fixing structure can not only effectively prevent unscrewing of the implantable medical device (e.g., a leadless pacemaker) away from the target organic tissue (e.g., myocardial tissue), but can also prevent the implantable medical device (e.g., a leadless pacemaker) from incrementally moving deeper into the target organic tissue (e.g., myocardial tissue) and finally penetrating through the tissue. Thus, higher reliability and increased safety can be obtained after the implantable medical device is implanted to the target organic tissue.

Since the implantable medical device is of the same inventive concept as the anti-reverse fixing structure, it has at least all the advantages of the anti-reverse fixing structure. For brevity, these advantages are not repeated here, and reference is made to the above description in connection with the anti-reverse fixing structure for more details.

### BRIEF DESCRIPTION OF THE DRAWINGS

Fig. 1 shows a schematic representation of a leadless pacemaker, in which an anti-reverse fixing structure provided herein is used.
Fig. 2 shows an exploded view of an anti-reverse fixing structure according to a first embodiment disclosed herein.
Fig. 3 schematically depicts a fixation helix of Fig. 2.
Fig. 4 schematically depicts a first support of Fig. 2.
Fig. 5 schematically depicts a first anti-reverse element of Fig. 2.
Fig. 6a shows an elevation view of the first anti-reverse element of Fig. 2, which is being subject to a first external force acting along an axis of the anti-reverse fixing structure from the distal end to the proximal end of the anti-reverse fixing structure.
Fig. 6b shows an elevation view of the first anti-reverse element of Fig. 2, which is being subject to a second external force acting along the axis of the anti-reverse fixing structure from the distal end to the proximal end of the anti-reverse fixing structure.
Fig. 6c shows an elevation view of the first anti-reverse element of Fig. 2, which is being subject to a third external force acting along the axis of the anti-reverse fixing structure from the distal end to the proximal end of the anti-reverse fixing structure.
Fig. 6d schematically depicts the first anti-reverse element of Fig. 6a, as viewed along the axis of the anti-reverse fixing structure from the distal end to the proximal end of the anti-reverse fixing structure.
Fig. 6e schematically depicts the first anti-reverse element of Fig. 6b, as viewed along the axis of the anti-reverse fixing structure from the distal end to the proximal end of the anti-reverse fixing structure.
Fig. 6f schematically depicts the first anti-reverse element of Fig. 6c, as viewed along the axis of the anti-reverse fixing structure from the distal end to the proximal end of the anti-reverse fixing structure.
Fig. 7 shows an exploded view of an anti-reverse fixing structure according to a second embodiment disclosed herein.
Fig. 8 schematically depicts a fixation helix and a first welding plate of Fig. 7.
Fig. 9 schematically depicts a first support of Fig. 7.
Fig. 10a shows an axial cross-sectional view of a first anti-reverse element of Fig. 7, which is being subject to a fourth external force acting along an axis of the anti-reverse fixing structure from the distal end to the proximal end of the anti-reverse fixing structure.
Fig. 10b shows an axial cross-sectional view of the first anti-reverse element of Fig. 7, which is being subject to a fifth external force acting along the axis of the anti-reverse fixing structure from the distal end to the proximal end of the anti-reverse fixing structure.
Fig. 10c shows an axial cross-sectional view of the first anti-reverse element of Fig. 7, which is being subject to a sixth external force acting along the axis of the anti-reverse fixing structure from the distal end to the proximal end of the anti-reverse fixing structure.
Fig. 10d schematically depicts the first anti-reverse element of Fig. 10a, as viewed along the axis of the anti-reverse fixing structure from the distal end to the proximal end of the anti-reverse fixing structure.
Fig. 10e schematically depicts the first anti-reverse element of Fig. 10b, as viewed along the axis of the anti-reverse fixing structure from the distal end to the proximal end of the anti-reverse fixing structure.
Fig. 10f schematically depicts the first anti-reverse element of Fig. 10c, as viewed along the axis of the anti-reverse fixing structure from the distal end to the proximal end of the anti-reverse fixing structure.
Fig. 11 shows an exploded view of an anti-reverse fixing structure according to a third embodiment disclosed herein.
Fig. 12 schematically depicts a fixation helix and a second welding plate of Fig. 11.
Fig. 13 schematically depicts a second anti-reverse element, the second welding plate and the fixation helix of Fig. 11.
Fig. 14a shows an elevation view of the second anti-reverse element of Fig. 11, which is being subject to a seventh external force acting along an axis of the anti-reverse fixing structure from the distal end to the proximal end of the anti-reverse fixing structure.
Fig. 14b shows an elevation view of the second anti-reverse element of Fig. 11, which is being subject to an eighth external force acting along the axis of the anti-reverse fixing structure from the distal end to the proximal end of the anti-reverse fixing structure.
Fig. 14c schematically depicts the second anti-reverse element of Fig. 14a, as viewed along the axis of the anti-reverse fixing structure from the distal end to the proximal end of the anti-reverse fixing structure.
Fig. 14d schematically depicts the second anti-reverse element of Fig. 14b, as viewed along the axis of the anti-reverse fixing structure from the distal end to the proximal end of the anti-reverse fixing structure.
Fig. 15 schematically depicts a mounting member of Fig. 11.
Fig. 16 shows an axial cross-sectional view of the fixation helix, the second welding plate, the second anti-reverse element and the mounting member of Fig. 11, which are integrally formed into one piece.
Fig. 17 shows an exploded view of an anti-reverse fixing structure according to a fourth embodiment disclosed herein.
Fig. 18 schematically depicts a fixation helix and a third anti-reverse element of Fig. 17.
Fig. 19 schematically depicts a second support of Fig. 17.
Fig. 20a shows an elevation view of the third anti-reverse element of Fig. 17, which is being subject to a ninth external force acting along an axis of the anti-reverse fixing structure from the distal end to the proximal end of the anti-reverse fixing structure.
Fig. 20b shows an elevation view of the third anti-reverse element of Fig. 17, which is being subject to a tenth external force acting along the axis of the anti-reverse fixing structure from the distal end to the proximal end of the anti-reverse fixing structure.
Fig. 20c schematically depicts the third anti-reverse element of Fig. 20a, as viewed along the axis of the anti-reverse fixing structure from the distal end to the proximal end of the anti-reverse fixing structure.
Fig. 20d schematically depicts the third anti-reverse element of Fig. 20b, as viewed along the axis of the anti-reverse fixing structure from the distal end to the proximal end of the anti-reverse fixing structure.
Fig. 21 shows an axial cross-sectional view of the fixation helix, the third anti-reverse element, the second support and a mounting member of Fig. 17, which are integrally formed into one piece.

### List of Reference Numerals

1 anti-reverse fixing structure; 2 electrical component; 3 power supply component; 4 housing; 5 distal docking member;
100 fixation substructure; 110 fixation helix; 111 variable pitch structure; 112 first welding surface; 120 connecting member; 121 ring-shaped base; 122 L-shaped coupling feature; 131 first welding plate; 1311 first locating feature; 1312 second welding surface; 1313 welding plate mounting feature; 132 second welding plate; 1321 third welding surface; 1322 second locating feature; 1323 first limiting feature;
200 anti-reverse substructure; 211 first support; 2111 groove feature; 2111a helix mounting feature; 2112 axial central feature; 2112a stepped support element; 2112b connecting shaft; 2113 second anti-reverse feature; 2114 side groove; 2115 bottom groove; 221 first anti-reverse element; 2211, 2221, 2231 first anti-reverse feature; 2211a, 2221a, 2223a first slanted surface; 2211b, 2221b, 2223b second slanted surface; 2211c, 2221c third slanted surface; 2211d fourth slanted surface; 2212 anti-reverse element mounting feature; 2212a engagement block;
222 second anti-reverse element; 2222 first anti-reverse base; 2223 third anti-reverse feature; 22221 first annular step; 22222 second limiting feature; 22223 second annular step; 22224 fourth limiting feature;
223 third anti-reverse element; 2232 second anti-reverse base; 22321 third annular step; 22322 third locating feature; 22323 fourth welding surface; 22324 fifth limiting feature;
212 second support; 2121 third anti-reverse base; 21211 fourth annular step; 21212 fifth annular step; 21214 sixth limiting feature; 21215 seventh limiting feature; 21213 support base; 2122 fourth anti-reverse feature; 2122a fifth slanted surface; 2122b sixth slanted surface;
300 mounting substructure; 310 mounting member; 311 passage hole; 312 third limiting feature; 320 sealing disc; 321 through hole;
400 electrode; 500 feedthrough; 600 feedthrough wire; 700 drug pill.

### DETAILED DESCRIPTION

Objects, advantages and features of the present application will become more apparent upon reading the following more detailed description of anti-reverse fixing structures and implantable medical devices proposed herein in conjunction with the accompanying drawings. Note that the figures are rather simplified and not necessarily to scale, with the only intention to help explain embodiments of the invention disclosed herein in a more convenient and clearer way. It will be understood that the figures may not necessarily illustrate the structures described herein to scale and that illustrative features depicted in the figures to explain certain principles of the present invention may be slightly simplified. The specific design features of the application as disclosed herein, including, for example, specific dimensions, orientations, locations and shapes, will be determined in part by the particular intended application and use environment. Additionally, in the embodiments described below, like reference numerals may be sometimes used to refer to the same or functionally similar elements throughout different figures, while description thereof may not be repeated. In this specification, similar reference numerals and letters refer to similar items in the figures, and thus once an item is defined in one figure, it may not be discussed for following figures. Where appropriate, the terms so used are interchangeable.

It is to be noted that, as used herein, the terms "center", "longitudinal", "transverse", "length", "width", "thickness", "up", "down", "front", "rear", "left", "right", "vertical", "horizontal", "top", "bottom", "inside", "outside", "clockwise", "counterclockwise", "axial", "radial", "circumferential", "axis" and so on may be used herein to describe orientations or positional relationships as viewed in the annexed figures. They are for convenience and ease of description of the present invention only and do not indicate or imply that the described apparatus or element must assume, or be constructed or operated in, a particular orientation. Therefore, they are not to be construed as limiting the present invention.

In addition, as used herein, the terms "first", "second" and the like are intended only for illustration and are not to be construed as denoting or implying relative importance, or as implicitly indicating the number of the referenced items. Accordingly, defining an item with "first" or "second" is an explicit or implicit indication of the presence of at least one such item. As used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements, unless otherwise clearly defined. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

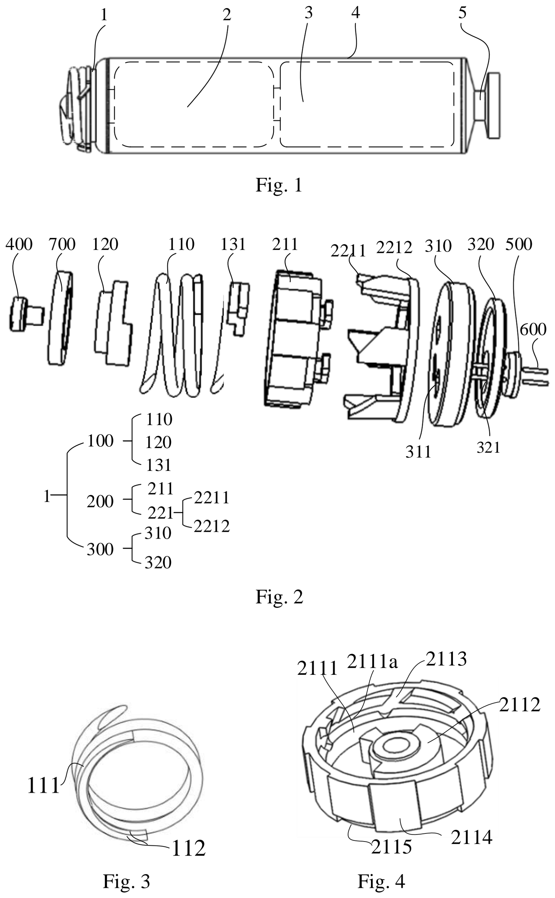

It is to be noted that, as used herein, the term "proximal end" refers to an end closer to an operator (and hence farther away from target tissue, such as target organic tissue), while the term "distal end" refers to an end farther away from the operator (and hence closer to the target organic tissue). In particular, anti-reverse fixing structures proposed herein can be used with various implantable medical devices. The present application is not limited to any particular type of implantable medical device, or to any particular implantation site. For example, such an implantable medical device may be any of a cardiac defibrillator, a cardiac pacemaker, a leadless pacemaker, an insulin pump, a drug delivery system, a brain neurostimulator and a monitoring system, or of another type than the enumerated examples. For ease of understanding, anti-reverse fixing structures proposed herein are described in the context of being used with leadless pacemakers as exemplary implantable medical devices in which they can be employed. In more detail, reference is made to Fig. 1, which schematically illustrates a leadless pacemaker incorporating an anti-reverse fixing structure proposed herein. As can be seen from Fig. 1, in the illustrated example, the leadless pacemaker includes the anti-reverse fixing structure 1, an electrical component 2 and a power supply component 3 both located in a housing 4, and a proximal docking member 5. Specifically, the anti-reverse fixing structure 1 is provided at a distal end of the leadless pacemaker primarily to fix the leadless pacemaker to myocardial tissue and prevent the leadless pacemaker from reversing and unscrewing. An electrode (not labeled in Fig. 1, but as 400 in Fig. 2, 7, 11 or 17) is connected to the electrical component 2 (more precisely, the electrode is electrically conductively welded to a feedthrough wire for the leadless pacemaker, which is fed through the anti-reverse fixing structure 1; it is to be noted that the electrode is fixedly coupled, but not electrically connected, to the anti-reverse fixing structure 1 and serves essentially to support and secure the electrode) to provide pacing and sensing. As a core component of the leadless pacemaker, the electrical component 2 serves essentially for overall control functions, such as communication with an external monitor and control of pacing and sensing of the electrode. The power supply component 3 is used to provide energy to the electrical component 2. The housing 4 is used to provide protection for the electrical component 2 and the power supply component 3. In addition, the housing 4 is generally provided thereon with a ring electrode (not labeled) for sensing activities of myocardial tissue. The proximal docking member 5 is mainly configured for docking with a delivery or retrieval system. In order to more clearly illustrate how the proposed anti-reverse fixing structure 1 is coupled in the leadless pacemaker, other components that the leadless pacemaker may have, in particular an electrode 400, a feedthrough 500, a feedthrough wire 600 and a drug pill 700, as shown in Fig. 2, 7, 11 or 17, may be mentioned in Examples 1 to 4 below. However, more detailed description of the electrode 400, the feedthrough 500, the feedthrough wire 600 and the drug pill 700 in the leadless pacemaker is omitted herein, and the details of them are well known to those skilled in the art to which this application pertains.

It is to be further noted that, for ease of understanding and brevity, the same or similar components commonly shared among anti-reverse fixing structures according to embodiments disclosed herein will be generally described first, and unique features of individual embodiments that are not owned by other embodiments will be then detailed in a few specific examples presented below. Additionally, numerical terms such as "at least one", "multiple", etc., when used herein as cardinal numbers to indicate quantity of something, are intended to mean that the quantity indicated by that numerical term may be plural (one or more) in accordance with the present application, without limiting the application, such as one, two, three or more, and may be appropriately determined depending on the actual circumstances in practical implementations of the application. For example, as used hereinafter, the phrase "at least one first anti-reverse feature" may be interpreted to mean that there may be one, two, three, or even more, such as seven or eight such first anti-reverse features.

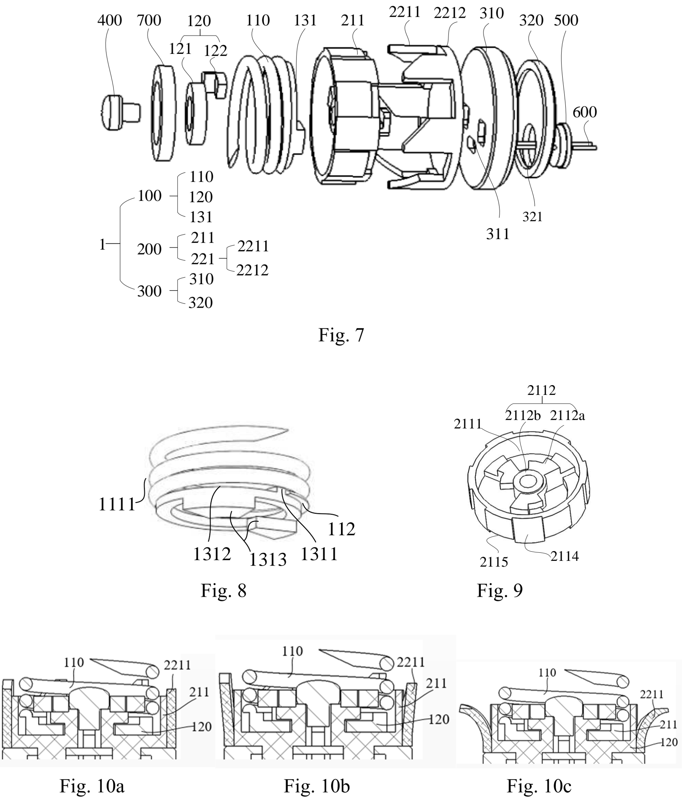

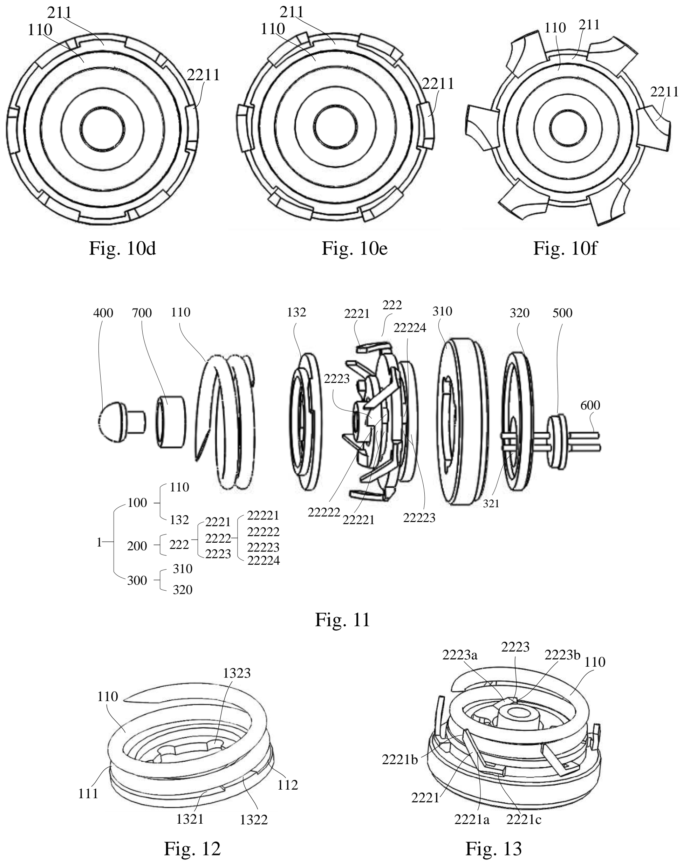

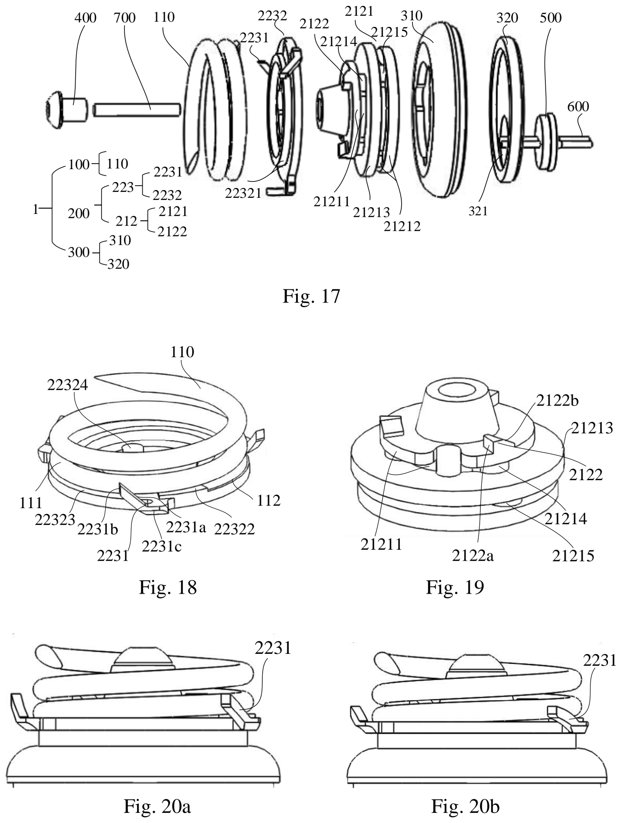

It is a principal object of the present application to provide an anti-reverse fixing structure 1 for use with an implantable medical device. For example, reference is made to Fig. 2, 7, 11 or 17. Fig. 2 shows an exploded view of an anti-reverse fixing structure 1 according to a first embodiment of the present application. Fig. 7 shows an exploded view of an anti-reverse fixing structure 1 according to a second embodiment of the present application. Fig. 11 shows an exploded view of an anti-reverse fixing structure 1 according to a third embodiment of the present application. Fig. 17 shows an exploded view of an anti-reverse fixing structure 1 according to a fourth embodiment of the present application. As can be readily seen from Fig. 2, 7, 11 or 17, each of the proposed anti-reverse fixing structures 1 includes a fixation substructure 100 for fixing an implantable medical device to target organic tissue, an anti-reverse substructure 200 and a mounting substructure 300 configured for coupling with a distal end of the implantable medical device. The fixation substructure 100 is connected to the mounting substructure 300 by the anti-reverse substructure 200. The anti-reverse substructure 200 includes at least one first anti-reverse feature (labeled as 2211 in the first and second embodiments, 2221 in the third embodiment, and 2231 in the fourth embodiment), which will deform, particularly bend, at its distal end so as to come into abutment, at least in part, with the target organic tissue, when stressed at the distal end by an external force acting along an axis of the anti-reverse fixing structure 1 from a distal end to a proximal end thereof.

As noted above, an anti-reverse fixing structure 1 is proposed herein, which includes a fixation substructure 100 for fixing an implantable medical device to target organic tissue, an anti-reverse substructure 200 and a mounting substructure 300 configured for connecting with a distal end of the implantable medical device. With this arrangement, not only the proposed anti-reverse fixing structure 1 can easily fix the implantable medical device to the target organic tissue, but a modular design is provided, which makes the anti-reverse fixing structure 1 simple in structure and easy to fabricate, assemble and maintain, resulting in manpower, material and time savings. Additionally, in the proposed anti-reverse fixing structure 1, the anti-reverse substructure 200 includes at least one first anti-reverse feature, which will bend at its distal end so as to come into abutment, at least in part, with the target organic tissue, when stressed at the distal end by an external force acting along an axis of the anti-reverse fixing structure 1 from a distal end to a proximal end thereof. With this arrangement, the proposed anti-reverse fixing structure 1 can not only effectively prevent the unscrewing of the implantable medical device (e.g., a leadless pacemaker) away from the target organic tissue (e.g., myocardial tissue), but can also prevent the implantable medical device (e.g., a leadless pacemaker) from incrementally moving deeper into the target organic tissue (e.g., myocardial tissue) and finally penetrating through the tissue. Thus, higher reliability and increased safety can be obtained after the implantable medical device is implanted to the target organic tissue.

It should be noted that the present application is not limited to any particular way in which the first anti-reverse feature bends. For example, in some embodiments, e.g., in the first and second embodiments described below, the first anti-reverse feature bends at the distal end radially outwards (i.e., away from the axis of the anti-reverse fixing structure in its radial direction perpendicular to the axis). In some other embodiments, e.g., in the third and fourth embodiments described below, the first anti-reverse feature bends at the distal end axially (i.e., along its axis) towards its proximal end. In yet some other embodiments, the first anti-reverse feature may bend at the distal end both radially outwards and axially towards the proximal end. This will be described in greater detail below, and no further description of it is necessary here.

Continued reference is made to Fig. 2, 7, 11 or 17. As can be readily seen from Fig. 2, 7, 11 or 17, according to some exemplary embodiments, an anti-reverse substructure 200 includes a plurality of first anti-reverse features (labeled as 2211 in the first and second embodiments, 2221 in the third embodiment and 2231 in the fourth embodiment), which are arranged around the axis of the anti-reverse fixing structure 1. Arranging the first anti-reverse features around the axis of the anti-reverse fixing structure 1 provides for outward bending of the first anti-reverse features at their respective distal ends in respective radial directions of the anti-reverse fixing structure 1 into at least partial abutment with the target organic tissue. Moreover, arranging the first anti-reverse features around the axis of the anti-reverse fixing structure 1 enables more uniform circumferential stressing of the anti-reverse substructure 200 during the outward bending of the first anti-reverse features, which can ensure that the implantable medical device can be maintained at a predetermined implantation site without deflections, thereby additionally enhancing the post-implantation reliability and safety of the implantable medical device.

Optionally, the first anti-reverse features may be made of a flexible biocompatible material and deformable so as to each have a reduced axial height and/or an increased axial projection. With this arrangement, the flexible biocompatible material of the first anti-reverse features ensures that the first anti-reverse features are easily deformable under the action of an external force (e.g., in the first and second embodiments, each first anti-reverse feature, when stressed at its distal end by an external force acting along the axis of the anti-reverse fixing structure 1 from the distal end to the proximal end thereof, can easily bend outwards in a radial direction of the anti-reverse fixing structure 1 so as to come into abutment, at least in part, with the target organic tissue). Moreover, the biocompatible material may be compatible with the target organic tissue and does not cause any immune reaction or rejection thereof, thereby ensuring good safety after the anti-reverse fixing structure 1 is implanted to the target organic tissue.

Continued reference is made to Fig. 2, 7, 11 or 17. As can be readily seen from Fig. 2, 7, 11 or 17, a fixation substructure 100 includes a fixation helix 110 for attachment to target organic tissue, the fixation helix 110 defines an axis in coincidence with the axis of the anti-reverse fixing structure 1. A proximal end of the fixation helix 110 is fixedly coupled to a distal end of the anti-reverse substructure 200. The axis of the anti-reverse fixing structure 1 is defined by a line connecting the proximal and distal ends of the anti-reverse fixing structure 1. With this design, during the implantation of implantable medical device to target organic tissue using the proposed anti-reverse fixing structure 1, the axis of the fixation helix 110 coincides with the axis of the anti-reverse fixing structure 1, and the proximal end of the fixation helix 110 is coupled (optionally, fixedly) to the distal end of the anti-reverse substructure 200. This not only allows for ease of fabrication and assembly, but also facilitates the implantation of an implantable medical device to target organic tissue using the proposed anti-reverse fixing structure 1.

In some optional embodiments, the fixation helix 110 is made of a biocompatible metallic material. In some other embodiments, the fixation helix 110 is provided with a non-metallic coating on part of its surface. With this arrangement, on the one hand, the biocompatible nature of the metallic material allows the fixation helix 110 to be compatible with target organic tissue, without causing any immune reaction or rejection, thereby ensuring good safety after the anti-reverse fixing structure 1 is implanted to the target organic tissue. On the other hand, the metallic material can impart excellent stability and durability to the fixation helix 110. Further, the use of the non-metallic coating can prevent the fixation helix 110 from being electrically connected to the myocardium, thereby enabling the fixation helix 110 to function as an antenna after the fixation helix 110 is electrically connected to the electrical component 2 by a feedthrough. Thus, it can be ensured that antenna functions are provided. Specifically, the materials of the biocompatible metal include, but are not limited to, stainless steel, nickel-titanium alloys, MP35N and 35NLT, which are commonly used metals for the active fixation helices 110 of electrode leads. The non-metallic coating on part of the surface of the fixation helix 110 may be made of a material including, but not limited to, Parylene C, polypropylene (PP), silicone, polyethylene (PE), polyurethane (PU), nylon, PU, polyimide (PI), polyether ether ketone (PEEK), polycarbonate (PC), etc.

### Example 1

In a first embodiment, there is provided an anti-reverse fixing structure 1. Specifically, reference is made to Fig. 2. As with what has been described earlier, in this embodiment, the anti-reverse fixing structure 1 includes a fixation substructure 100, which in turn includes a fixation helix 110. The fixation helix 110 may be the same as what has been described in the preceding paragraphs, and no further description of it is necessary here. In addition to the fixation helix 110, the fixation substructure 100 in the anti-reverse fixing structure 1 in this embodiment further includes a first welding plate 131. A distal end of the first welding plate 131 (i.e., the left-hand end thereof as viewed in the orientation of Fig. 2) is coupled (optionally, fixedly) to a proximal end of the fixation helix 110 (i.e., the right-hand end thereof as viewed in the orientation of Fig. 2). A proximal end of the first welding plate 131 (i.e., the right-hand end thereof as viewed in the orientation of Fig. 2) is coupled (optionally, fixedly) to a distal end of an anti-reverse substructure 200 (i.e., the left-hand end thereof as viewed in the orientation of Fig. 2). In an optional implementation, the proximal end of the first welding plate 131 may be coupled (optionally, fixedly) to a first support 211. With this arrangement, through fixedly coupling the fixation helix 110 to the first welding plate 131, and the first welding plate 131 to the distal end of the anti-reverse substructure 200, an implantable medical device (e.g., a leadless pacemaker) can be fixed to target organic tissue at a predetermined site (e.g., a specified myocardial site, such as the apex, interventricular septum, right atrial appendage, smooth muscle, etc.) and made easy to fabricate and assemble. Moreover, higher coupling strength can be obtained between the fixation helix 1110 and the first support 211. Further, the implantable medical device can be easily mechanically coupled and electrically connected to one of feedthrough wires 600 in a feedthrough 500. It should be noted that the present application is not limited to any particular way in which the fixation helix 110 is coupled to the first welding plate 131. For example, the coupling of the fixation helix 110 and the first welding plate 131 may be accomplished by laser welding.

Optionally, the first welding plate 131 may be made of a biocompatible metallic material. The biocompatible material may be compatible with the target organic tissue and does not cause any immune reaction or rejection thereof, providing for high safety after the anti-reverse fixing structure 1 is implanted to the target organic tissue. In addition, the metallic material allows the first welding plate 131 to be more easily fixedly coupled (e.g., welded) to the fixation helix 110 and to provide better support for the fixation helix 110, thus enhancing axial stability of the fixation helix 110. Particular examples of the biocompatible metallic material may include, but are not limited to, stainless steel, nickel-titanium alloys, MP35N and 35NLT.

Reference is now made to Fig. 2. As can be seen from Fig. 2, in some exemplary implementations, the fixation substructure 100 further includes a connecting member 120, the connecting member 120 is disposed at least partially inside the fixation helix 110 along an axis of the anti-reverse fixing structure 1. A distal end of the connecting member 120 (i.e., the left-hand end thereof as viewed in the orientation of Fig. 2) is configured to secure an electrode 400 of the implantable medical device. A proximal end of the connecting member 120 (i.e., the right-hand end thereof as viewed in the orientation of Fig. 2) is coupled (optionally, fixedly) to the distal end of the anti-reverse substructure 200. In an optional implementation, the proximal end of the connecting member 120 may be coupled (optionally, fixedly) to the first support 211. With this arrangement, in the anti-reverse fixing structure 1 of this embodiment, the connecting member 120 included in the fixation substructure 100 can not only well support the electrode 400 and a drug pill 700, but can also assist in blocking movement of the first welding plate 131 along the axis of the anti-reverse fixing structure 1, thereby enabling the first welding plate 131 to be even more securely coupled to the fixation helix 110 and the anti-reverse substructure 200 (in an optional implementation, the proximal end of the fixation helix 110 may be fixedly coupled to the first support 211). Optionally, the connecting member 120 may be made of a biocompatible non-metallic material. Particular examples of the biocompatible non-metallic material may include, but are not limited to, PP, silicone, PE, PU, nylon, PU, PI, PEEK and PC. As detailed blow, the connecting member 120 and the first support 211 may be fixedly coupled to each other by applying a curable resin adhesive to them after they are brought into correct contact with each other.

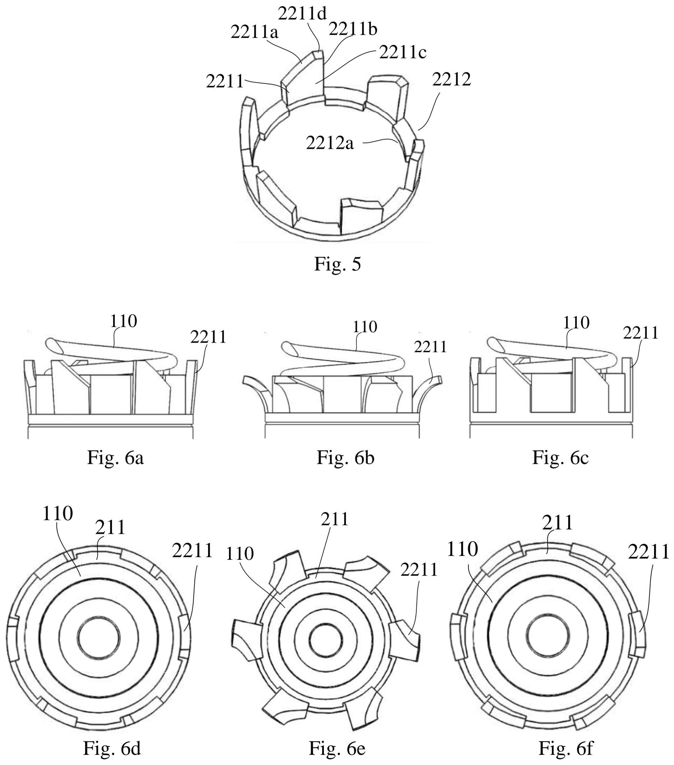

Reference is made to Figs. 2, 3, 4 and 5. Fig. 3 shows a schematic representation of the fixation helix 110 of Fig. 2. Fig. 4 shows a schematic representation of the first support 211 of Fig. 2. Fig. 5 shows a schematic representation of a first anti-reverse element 221 of Fig. 2. As can be seen from Figs. 2, 3, 4 and 5, in some exemplary implementations, the anti-reverse substructure 200 includes a first anti-reverse element 221 (see Fig. 5) in circumferential engagement with the first support 211 (see Fig. 4). A distal end of the first support 211 is coupled (optionally, fixedly) to part of the fixation helix 110 and the proximal end of the connecting member 120, and a proximal end of the first support 211 is coupled (optionally, fixedly) to a distal end of a mounting substructure 300. The first anti-reverse element 221 includes a first anti-reverse feature 2211 and an anti-reverse element mounting feature 2212. The first anti-reverse feature 2211 is provided on the anti-reverse element mounting feature 2212.

Optionally, the first support 211 may be made of a biocompatible non-metallic material, such as PP, silicone, PE, PU, nylon, PU, PI, PEEK or PC. With this arrangement, the biocompatible non-metallic material allows the first support 211 to be more easily fixedly coupled to the first welding plate 131 and the connecting member 120, for example, by applying a curable resin adhesive to them after they are brought into correct contact with each other. During assembly, the first welding plate 131 may be seated in the first support 211, and the fixation helix 110 may be then mounted to a securing feature in the first support 211 (e.g., a helix mounting feature 2111a as described below) and welded to the first welding plate 131. After that, the connecting member 120 may be fixedly coupled to the first support 211.

As noted above, in some exemplary implementations of this embodiment, the first anti-reverse feature 2211 is bendable at its distal end so as to come into abutment, at least in part, with the target organic tissue. Such bendability includes radially outward bendability of the first anti-reverse feature 2211 at its distal end into abutment, at least in part, with the target organic tissue.

Specifically, in some exemplary implementations, with continued reference to Figs. 2 and 4, the first support 211 is formed therein with a side groove 2114 and a bottom groove 2115. The first anti-reverse feature 2211 is aligned in position with and engaged in the side groove 2114, and the anti-reverse element mounting feature 2212 is positioned in the bottom groove 2115 and sandwiched between the first support 211 and the mounting substructure 300. With this design, the first anti-reverse feature 2211 is engaged in the side groove 2114 of the first support 211, and the anti-reverse element mounting feature 2212 is positioned in the bottom groove 2115 and sandwiched between the first support 211 and the mounting substructure 300, in the anti-reverse fixing structure 1 of this embodiment. This not only enables the first anti-reverse feature 2211 to be more securely coupled to the first support 211 along the axis of, and radially with respect to (i.e., perpendicular to the axis; the same applies below), the anti-reverse fixing structure 1, and further facilitates outward bending of the first anti-reverse feature 2211 at its distal end in a radial direction of the anti-reverse fixing structure 1 under the action of an external force applied to the distal end along the axis of the anti-reverse fixing structure 1 from the distal end to the proximal end of the anti-reverse fixing structure.

Referring to Figs. 2, 4 and 5, in some exemplary implementations, the first anti-reverse feature 2211 extends in a direction pointing from a proximal end of the first anti-reverse element 221 to a distal end thereof, and the anti-reverse element mounting feature 2212 is in the form of an annulus fitted in the bottom groove 2115 of the first support 211. Through fitting the annulus in the bottom groove 2115, the first anti-reverse element 221 is restrained from translation along the axis of the anti-reverse fixing structure 1. Moreover, through engaging the first anti-reverse feature 2211 in the side groove 2114, the anti-reverse element 221 is also restrained from circumferential rotation. As a result, the first anti-reverse element 221 can be more securely coupled to the first support 211, further facilitating the arrangement of the first anti-reverse feature 2211 around the axis of the anti-reverse fixing structure 1.

With continued reference to Fig. 5, in some exemplary implementations, the anti-reverse element mounting feature 2212 is provided with engagement blocks 2212a, the engagement blocks 2212a are spaced on an inner surface thereof and project inwards perpendicularly to the axis of the anti-reverse fixing structure 1. In the bottom groove 2115, respective mating engagement grooves (not shown) are formed at locations corresponding to the respective engagement blocks 2212a. The engagement blocks 2212a can project into the respective engagement grooves. With this arrangement, engaging the engagement blocks 2212a in the respective engagement grooves not only facilitates assembly, but also allows the first anti-reverse element 221 to be more securely coupled to the first support 211.

With continued reference to Figs. 2 and 4, in some exemplary implementations, a groove feature 2111 is formed at the distal end of the first support 211, and the helix mounting feature 2111a is formed in an inner wall surface of the groove feature 2111 so that the fixation helix 110 can be partially retained in the helix mounting feature 2111a. With this arrangement, forming the groove feature 2111 at the distal end of the first support 211, on the one hand, provides for forming the helix mounting feature 2111a on the inner wall surface of the groove feature 2111 and, on the other hand, the arrangement of the groove feature 2111 also establishes the basis for the first support 211 to accommodate the first welding plate 131 and the connecting member 120. This not only enables the first support 211 to provide better support, but also allows the anti-reverse fixing structure 1 to be made with an effectively reduced axial dimension. Furthermore, through providing the helix mounting feature 2111a, not only the fixation helix 110 can be made easier to assemble, but deformability of the fixation helix 110 can be also effectively limited to deformability along the axis of the anti-reverse fixing structure 1, thereby effectively reducing the risk of the target organic tissue (e.g., myocardial tissue) being penetrated through (e.g., due to excessive axial deformation of the fixation helix 110).

Optionally, with continued reference to Figs. 2 and 4, the helix mounting feature 2111a may be a helical groove complementary in shape to the portion of the fixation helix 110 retained therein. With this design, retaining the portion of the fixation helix 110 in the complementary helical groove can ensure that the portion of the fixation helix 110 retained in the helical groove is held stable with respect to the first support 211, thereby enabling more reliable deformability of the fixation helix 110 along the axis of the anti-reverse fixing structure 1.

With continued reference to Fig. 4, in some exemplary implementations, the first support 211 further includes at least one second anti-reverse feature 2113, which is provided in the inner wall surface of the groove feature 2111 and brought into communication with the helix mounting feature 2111a. The second anti-reverse feature 2113 is a groove, which is open both on the same side as the helical groove is open and at its distal end. Through providing at least one second anti-reverse feature 2113 in the form of a groove in the inner wall surface of the groove feature 2111, the second anti-reverse feature 2113 is in communication with the helix mounting feature 2111a and open both on the same side as the helical groove is open and at its distal end, the fixation helix 110 can be additionally restricted and prevented from unscrewing away from the target organic tissue. In particular, the fixation helix 110 is generally made of a biocompatible metallic material, while the first support 211 is generally made of biocompatible non-metallic material. Through providing the second anti-reverse feature 2113 in the form of an open groove, after the device is implanted to the target organic tissue, the softer target organic tissue will enter and be retained in the grooved second anti-reverse feature 2113 while abutting against its open end, thereby preventing unscrewing.

Optionally, the fixation helix 110 may be a variable pitch structure 111. This design with a varying pitch can not only facilitate assembly of the fixation helix 110 with the helix mounting feature 2111a (in the shape of a helical groove), but can also effectively reduce the risk of the target organic tissue being penetrated through. Specifically, the fixation helix 110 may be designed with a greater pitch around its distal end than around its proximal end. With this arrangement, the fixation helix is allowed to have an even shorter length while achieving desirable fixation without causing myocardial perforation.

Continued reference is made to Fig. 4. In some exemplary implementations, as can be seen from Fig. 4, the first support 211 further includes an axial central feature 2112 provided at the bottom of the groove feature 2111, the axial central feature 2112 is complementary in shape to the proximal end of the connecting member 120 and a welding plate mounting feature 1313 provided at the proximal end of the first welding plate 131 in the fixation substructure 100 (as can be particularly seen from Fig. 8). The axial central feature 2112 is provided to couple (optionally, fixedly) the connecting member 120 and the first welding plate 131 to the first support 211. Through providing the axial central feature 2112, not only the first support 211 can be more easily fixedly coupled to the connecting member 120 and the first welding plate 131, but the first support 211 is enabled to provide even better support. Further, this design allows the wall of the groove feature 2111 in the first support 211, the fixation helix 110, the connecting member 120, the first welding plate 131 and the axial central feature 2112 to be mutually nested from the outside inwards, thereby ensuring that the first support 211 provides good support, while enabling the anti-reverse fixing structure 1 to have an effectively reduced overall size. Thus, patient discomfort caused by the implantable medical device can be effectively reduced, and resource savings can be achieved.

Continued reference is made to Fig. 5. In some exemplary implementations, as can be seen from Fig. 5, the first anti-reverse feature 2211 is a plate-like structure defining a first slanted surface 2211a forming a first angle, a second slanted surface 2211b forming a second angle, and a third slanted surface 2211c forming a third angle, with a cross-section of the anti-reverse fixing structure 1. The first slanted surface 2211a is located at an end of the plate-like structure away from the anti-reverse element mounting feature 2212 and slanted in a direction (from the lower left corner toward the upper right corner, as viewed in the orientation of Fig. 5) opposite to an unscrewing direction for the fixation helix 110 (distally from upper left to lower right, as viewed in the orientation of Fig. 3). Both the second slanted surface 2211b and the third slanted surface 2211c extend from a proximal end of the plate-like structure to its distal end, and the third slanted surface 2211c is contiguous with both the first slanted surface 2211a and the second slanted surface 2211b. The first, second and third angles are all non-zero. The first angle is smaller than the second angle, and the third angle is smaller than 90°.

With this arrangement, the plate-like design of the first anti-reverse feature 2211 not only enables ease of fabrication, and allows the first anti-reverse feature 2211 to be received within the side groove 2114 of the first support 211 when not stressed by any external force. As noted above, the first slanted surface 2211a is located at the end of the plate-like structure away from the anti-reverse element mounting feature 2212 and slanted in a direction opposite to the unscrewing direction for the fixation helix 110. Moreover, the first, second and third angles are all non-zero, and the first angle is smaller than the second angle. Specifically, referring to Figs. 2 and 5 and imagining that you are holding your right hand with the thumb pointing to the left, as viewed in the orientation of Fig. 2 (i.e., toward the distal end of the anti-reverse fixing structure 1), and the other four fingers making a fist, the fixation helix 110 can be screwed to axially move deeper into the target organic tissue as a result of being turned in the direction in which the other four fingers are curled. Since the first angle between the first slanted surface 2211a and the cross-section of the anti-reverse fixing structure 1 is smaller than the second angle between the second slanted surface 2211b and the cross-section of the anti-reverse fixing structure 1, the fixation helix 110 will encounter greater resistance when it is turned in an opposite unscrewing direction (e.g., if you are holding your right hand with the thumb pointing from the right to the left, as viewed in the orientation of Fig. 2, and the other four fingers making a fist, then this unscrewing direction will be opposite to the direction in which the other four fingers are curled from the roots to the tips) than when it is turned in a screwing direction (opposite to the above described unscrewing direction). In this way, unscrewing of the fixation helix 110 can be prevented, and therefore the proposed anti-reverse fixing structure 1 has anti-reverse capabilities and can fix the implantable medical device securely to the target organic tissue. Further, since the third angle is smaller than 90°, the first anti-reverse feature 2211, when urged by the target organic tissue along the axis of the anti-reverse fixing structure 1, will tend to bend outwardly in a radial direction thereof while abutting against the target organic tissue. This results in a larger contact area between the first anti-reverse feature 2211 and the target organic tissue (e.g., myocardial tissue) and increased resistance to deeper penetration of the implantable medical device (e.g., a leadless pacemaker) as a whole into the target organic tissue along its axis, reducing the risk of the target organic tissue being penetrated through.

For example, the plate-like structure may be a flat plate, and a mounting member 310 may be made of pure titanium. In particular, the first angle between the first slanted surface 2211a and the cross-section of the anti-reverse fixing structure 1 (which may be an angle between the first slanted surface 2211a and a horizontal plane, as viewed in the orientation of Fig. 5) may be selected from the range of 30° to 60°. The second angle between the second slanted surface 2211b and the cross-section of the anti-reverse fixing structure 1 (which may be an angle between the second slanted surface 2211b and a horizontal plane, as viewed in the orientation of Fig. 5) may be selected from the range of 60° to 90°. It should be noted that the present application is not limited to any particular value of the first, second or third angle, as would be appreciated by those skilled in the art.

In some other implementations, the plate-like structure may be a curved plate, which is, however, not further described herein for the sake of brevity.

Optionally, with continued reference to Fig. 5, the first anti-reverse feature 2211 may further define a fourth slanted surface 2211d, the fourth slanted surface 2211d is contiguous with the first slanted surface 2211a, the second slanted surface 2211b and the third slanted surface 2211c, the fourth slanted surface 2211d defines a distal end of the plate-like structure together with the first slanted surface 2211a. The first slanted surface 2211a may extend a greater length circumferentially around the anti-reverse element mounting feature 2212 than the fourth slanted surface 2211d. Through providing the first slanted surface 2211a, the second slanted surface 2211b, the third slanted surface 2211c and the fourth slanted surface 2211d, unscrewing of the fixation helix 110 can be prevented, and the risk of the target organic tissue being penetrated through can be effectively lowered. Meanwhile, discomfort caused to the target organic tissue by a sharp corner, which would be otherwise present at the distal end of the first anti-reverse feature 2211 if the first slanted surface 2211a were directly contiguous with the second slanted surface 2211b without any transitions therebetween, can be avoided.

It should be noted that the present application is not limited to any particular number of first anti-reverse features 2211, and in practical implementations of the present application, any appropriate number of first anti-reverse features 2211 may be included according to the material used and torque test results while taking into account constraints resulting from dimensions of the anti-reverse fixing structure 1. Optionally, the number of first anti-reverse features 2211 may range from two to eight. With this arrangement, the risk of the target organic tissue being penetrated through can be reduced, while taking into account easy fabrication and efficient assembly. In another optional implementation, the first anti-reverse element 221 may be an integral structure, which enables even more efficient assembly.

More specifically, reference is made to Figs. 6a to 6f. Fig. 6a shows an elevation view of the first anti-reverse element 221 of Fig. 2, which is being subject to a first external force acting along the axis of the anti-reverse fixing structure 1 from the distal end to the proximal end of the anti-reverse fixing structure. Fig. 6b shows an elevation view of the first anti-reverse element 221 of Fig. 2, which is being subject to a second external force acting along the axis of the anti-reverse fixing structure 1 from the distal end to the proximal end of the anti-reverse fixing structure. Fig. 6c shows an elevation view of the first anti-reverse element 221 of Fig. 2, which is being subject to a third external force acting along the axis of the anti-reverse fixing structure 1 from the distal end to the proximal end of the anti-reverse fixing structure. Fig. 6d schematically illustrates the first anti-reverse element of Fig. 6a, as viewed along the axis of the anti-reverse fixing structure 1 from the distal end to the proximal end of the anti-reverse fixing structure. Fig. 6e schematically illustrates the first anti-reverse element of Fig. 6b, as viewed along the axis of the anti-reverse fixing structure 1 from the distal end to the proximal end of the anti-reverse fixing structure. Fig. 6f schematically illustrates the first anti-reverse element of Fig. 6c, as viewed along the axis of the anti-reverse fixing structure 1 from the distal end to the proximal end of the anti-reverse fixing structure. From Figs. 6a to 6f, bending of the first anti-reverse feature 2211 under the action of the different external forces (the first external force is smaller than the second external force, which is in turn smaller than the third external force) can be seen, which occurs outwardly in a radial direction of the anti-reverse fixing structure 1 and enlarges a radial contour of the first anti-reverse element 221 (and thus results in an increased radial contact area between the first anti-reverse element 221 and the target organic tissue). Also, how the first support 211 is coupled to, and located relative to, the fixation helix 110 and the first anti-reverse element 221 can be clearly seen. This additionally demonstrates that the presence of the first support 211 can not only ensure that the fixation helix 110, the first welding plate 131, the connecting member 120 and the first anti-reverse element 221 are coupled together securely, but can also effectively prevent the fixation helix 110 from excessive radial deformation.

Continued reference is made to Fig. 2. As can be seen from Fig. 2, in some exemplary implementations, the mounting substructure 300 includes a mounting member 310 with a passage hole 311, and the proximal end of the first support 211 is inserted into the passage hole 311 and is thereby coupled (optionally fixedly; without limitation, such fixed coupling may be accomplished by filling a curable resin adhesive into a clearance between them) to the mounting member 310. Through fixedly coupling the first support 211 to the mounting member 310 by inserting its proximal end into the passage hole 311, issues associated with connection between non-metallic (the first support 211 is typically made of a non-metallic material) and metallic (the mounting member 310 is typically made of a metallic material) materials can be well circumvented.

Optionally, the first support 211 may be provided at its proximal end with at least one rod-like projection complementary in shape to the passage hole 311 in the mounting member 310. The rod-like projection may be inserted into the complementary passage hole 311, and a more secure coupling may be obtained between the two by filling a curable resin adhesive into a clearance between them, or otherwise. Optionally, the rod-like projection may have a proximal bent or curved portion, which may be passed through the passage hole 311 in the mounting member 310 and then brought into abutment with a proximal end face of the mounting member 310. This enables the first support 211 and the mounting member 310 to be even more securely coupled to each other. Additionally, it will be understood that, apart from the passage hole 311 for coupling with the first support 211, the mounting member 310 may also have a through hole (not labeled) for passage of a feedthrough wire 600 therethrough, which is, however, not further described herein. There may be a plurality of rod-like projections, optionally two rod-like projections.

In particular, the mounting member 310 may be made of pure titanium and thereby can be more easily welded to another component (e.g., a housing 4) of the implantable medical device (e.g., a leadless pacemaker). This can not only ensure that the anti-reverse fixing structure 1 is securely coupled to the other component of the implantable medical device, but can also provide sealing and protection for the feedthrough wire 600.

Continued reference is made to Fig. 2. As can be seen from Fig. 2, in some exemplary implementations, the mounting substructure 300 further includes a sealing disc 320 with a through hole 321. The sealing disc 320 is coupled (optionally, fixedly) to a proximal end of the mounting substructure 300, and the through hole 321 is configured for passage of the feedthrough wire 600 therethrough. With this arrangement, the sealing disc 320 can provide sealing for the implantable medical device.

### Example 2

In a second embodiment, there is provided an anti-reverse fixing structure 1. As mentioned earlier, the anti-reverse fixing structure 1 of this embodiment is as shown in Fig. 7. As can be seen from Fig. 7, the anti-reverse fixing structure 1 of this embodiment is substantially similar to the anti-reverse fixing structure 1 of the first embodiment and also includes a fixation substructure 100, an anti-reverse substructure 200 and a mounting substructure 300. The fixation substructure 100 also includes a fixation helix 110, a connecting member 120 and a first welding plate 131, and the anti-reverse substructure 200 also includes a first support 211 and a first anti-reverse element 221. The mounting substructure 300 also includes a mounting member 310 and a sealing disc 320. To avoid repetition, the previously described features of the anti-reverse fixing structure 1 of the first embodiment that are shared with this embodiment, such as the connecting member 120, the anti-reverse substructure 200, the mounting member 310, the sealing disc 320 and the materials of the components (e.g., the first welding plate 131 may be optionally made of a biocompatible metallic material; the first support 211 may be optionally made of a biocompatible non-metallic material; and so forth) are not repeated here. For more details of any of those features that help appropriately understand the specific feature, reference is made to the above description of the first embodiment in connection with the feature. Only differences from the first embodiment are described below.

First of all, in the anti-reverse fixing structure 1 of this embodiment, the fixation helix 110 is coupled to the first welding plate 131 in a different manner from that of the first embodiment. In particular, reference is made to Fig. 8, which schematically illustrates the fixation helix 110 and the first welding plate 131 of Fig. 7. A first welding surface 112 is provided at a proximal end of the fixation helix 110 (i.e., the lower end of the fixation helix 110 as viewed in the orientation of Fig. 8), and at a distal end of the first welding plate 131 (i.e., the upper end of the first welding plate 131 as viewed in the orientation of Fig. 8), there is provided a second welding surface 1312 matching the first welding surface 112. The first welding surface 112 is attached (optionally, fixedly) to the second welding surface 1312. Through providing the first welding surface 112 at the proximal end of the fixation helix 110 and the corresponding second welding surface 1312 at the distal end of the first welding plate 131, the fixation helix 110 can be more easily coupled to the first welding plate 131. For example, the fixation helix 110 may be fixedly coupled to the first welding plate 131 by attaching the first welding surface 112 to the second welding surface 1312 using laser welding. It should be noted that the present application is not limited to any type of welding for attaching the first welding surface 112 to the second welding surface 1312, and other types of welding than laser welding are also possible, such as gas welding, arc welding, electron-beam welding, friction welding and ultrasonic welding.

Optionally, the first welding plate 131 may further include a first locating feature 1311 for orienting the fixation helix 110 in a predetermined helical orientation. The presence of the first locating feature 1311 enables the first welding surface 112 of the fixation helix 110 to be welded to the second welding surface 1312 so that the fixation helix 110 is oriented in the predetermined helical orientation. Through orienting the fixation helix 110 in the desired helical orientation with the aid of the first locating feature 1311, not only the fixation helix 110 can be more easily and quickly assembled with the first welding plate 131, but it can also be ensured that the fixation helix 110 is fixedly coupled to the first welding plate 131 in a predetermined helical orientation. Optionally, the first locating feature 1311 may be a bump-like protrusion. With this arrangement, in order to weld the fixation helix 110 to the first welding plate 131, the end of the fixation helix 110 with the first welding surface 112 may be first brought into abutment with a side of the first locating feature 1311 facing toward the second welding surface 1312 (i.e., the right-hand side of the first locating feature 1311 as viewed in the orientation of Fig. 8), and the first welding surface 112 may be then welded to the second welding surface 1312 so that the fixation helix 110 has the aforementioned helical orientation. In this way, the first welding surface 112 can be attached to the second welding surface 1312 by welding so that the fixation helix 110 is oriented in the predetermined helical orientation.

In some exemplary implementations, the first welding plate 131 further includes a welding plate mounting feature 1313 provided at the proximal end of the first welding plate 131 (i.e., the lower end of the first welding plate 131 as viewed in the orientation of Fig. 8). The welding plate mounting feature 1313 is complementary in shape, and coupled (optionally, fixedly), to a distal end of the anti-reverse substructure 200 (more precisely, to an axial central feature 2112 of the first support 211). With this arrangement, the presence of the welding plate mounting feature 1313 enables the first welding plate 131 to be more easily and quickly assembled with the anti-reverse substructure 200 while ensuring that the first welding plate 131 is fixedly coupled to the anti-reverse substructure 200 at a predetermined location so that an unscrewing direction for the fixation helix 110 is opposite to a direction in which a first slanted surface 2211a of a first anti-reverse feature 2211 is slanted. For example, the welding plate mounting feature 1313 may be a first bump-like feature, which is raised toward a proximal end of the anti-reverse fixing structure 1 and extends inwardly in a radial direction thereof. Specifically, reference is made to Fig. 9, which schematically depicts the first support 211 of Fig. 7. As can be seen from Fig. 9, optionally, there may be two first bump-like features spaced apart from each other at a distance matching a distance between adjacent stepped support elements 2112a (as detailed below) of the axial central feature 2112 in the first support 211 of Fig. 9. On sides of the stepped support elements 2112a, there are provided second bump-like features (although they are not labeled in Fig. 9, an example of them is visible as a raised bump located to the left of an endpoint of the line leading from "2112a") complementary in shape to the first bump-like features. In order to secure the first welding plate 131 to the first support 211, the first bump-like features may be engaged in spaces delimited by the second bump-like features and the bottom of a groove feature 2111 in the first support 211, with one side face of each first bump-like feature (i.e., each welding plate mounting feature 1313) being brought into abutment with a side face of a lower portion of a stepped support element 2112a below a second bump-like feature. Thus, the first and second bump-like features provide for mutually nested engagement, which enables the first welding plate 131 and the first support 211 to be axially coupled to each other more securely.

Further, in the anti-reverse fixing structure 1 of this embodiment, the first support 211 and the fixation helix 110 are coupled in a different manner than that of the first embodiment. With continued particular reference to Fig. 9, in some exemplary implementations, differing from the first support 211 in the anti-reverse fixing structure 1 of the first embodiment (as schematically illustrated in Fig. 4), the axial central feature 2112 in the first support 211 of this embodiment includes at least one stepped support element 2112a extending from a central axis of the first support 211 radially toward a side wall of the first support 211. Each stepped support element 2112a includes a first step (not labeled) closer to the side wall and a second step (not labeled) closer to the central axis. The first step is below the second step. With this arrangement, the side wall of the first support 211 and the second step can confine the fixation helix 110 on the first step between the side wall of the first support 211 and the second step. On the one hand, this ensures stable engagement of the first support 211 with the portion of the fixation helix 110 therein. On the other hand, this enables the fixation helix 110 to deform along an axis of the anti-reverse fixing structure 1 in an even more reliable style and have an effectively reduced overall size. Optionally, there may be three stepped support elements 2112a equidistantly arranged within the groove feature 2111 of the first support 211. Apparently, the present application is not so limited, because in alternative implementations, there may be one, two, four or even more stepped support elements 2112a. In the case of more than one stepped support element 2112a, they may be spaced apart from one another to additionally limit unscrewing of the fixation helix 110 away from target organic tissue. Continued reference is made to Figs. 7 and 9. In Fig. 9, the axial central feature 2112 of the first support 211 further includes a connecting shaft 2112b positioned at the center of the axial central feature 2112. In Fig. 7, the connecting member 120 includes a ring-shaped base 121 and an L-shaped coupling feature 122 disposed at a proximal end of the ring-shaped base 121 (i.e., the right-hand end of the connecting member 120, as viewed in the orientation of Fig. 7). The ring-shaped base 121 has an inner diameter matching an outer diameter of the connecting shaft 2112b. The L-shaped coupling feature 122 is coupled at a top end thereof (i.e., the left-hand end of the L-shaped coupling feature 122, as viewed in the orientation of Fig. 7) to the ring-shaped base 121 and radially extends outwards so as to partially protrude beyond the ring-shaped base 121. The L-shaped coupling feature 122 defines a direction of bending, which is opposite to the helical orientation of the fixation helix 110. In order to secure the connecting member 120 to the first support 211, the ring-shaped base 121 may be disposed over the connecting shaft 2112b, with a bottom portion of the L-shaped coupling feature 122 being engaged between a second bump-like feature on a stepped support element 2112a and the bottom of the groove feature 2111 in the first support 211. With this arrangement, the direction of bending of the L-shaped coupling feature 122 that is opposite to the helical orientation of the fixation helix 110 and the mutually nested engagement of the L-shaped coupling feature 122 and a second bump-like feature not only allow the connecting member 120 to be axially coupled to the first support 211 more securely, but can also limit circumferential rotation of the connecting member and hence unscrewing of the fixation helix 110 away from the target organic tissue.

Specifically, reference is made to Figs. 10a to 10f. Fig. 10a shows an axial cross-sectional view of the first anti-reverse element 221 of Fig. 7, which is being subject to a fourth external force acting along the axis of the anti-reverse fixing structure 1 from the distal end to the proximal end of the anti-reverse fixing structure 1. Fig. 10b shows an axial cross-sectional view of the first anti-reverse element 221 of Fig. 7, which is being subject to a fifth external force acting along the axis of the anti-reverse fixing structure 1 from the distal end to the proximal end of the anti-reverse fixing structure 1. Fig. 10c shows an axial cross-sectional view of the first anti-reverse element 221 of Fig. 7, which is being subject to a sixth external force acting along the axis of the anti-reverse fixing structure 1 from the distal end to the proximal end of the anti-reverse fixing structure 1. Fig. 10d schematically depicts the first anti-reverse element 221 of Fig. 10a, as viewed along the axis of the anti-reverse fixing structure 1 from the distal end to the proximal end of the anti-reverse fixing structure 1. Fig. 10e schematically depicts the first anti-reverse element 221 of Fig. 10b, as viewed along the axis of the anti-reverse fixing structure 1 from the distal end to the proximal end of the anti-reverse fixing structure 1. Fig. 10f schematically depicts the first anti-reverse element 221 of Fig. 10c, as viewed along the axis of the anti-reverse fixing structure 1 from the distal end to the proximal end of the anti-reverse fixing structure 1. From Figs. 10a to 10f, bending of the first anti-reverse feature 2211 under the action of the different external forces (the fourth external force is smaller than the fifth external force, which is in turn smaller than the sixth external force) can be seen, which occurs outwardly in a radial direction of the anti-reverse fixing structure 1 and enlarges a radial contour of the first anti-reverse element 221 (and thus results in an increased radial contact area between the first anti-reverse element 221 and the target organic tissue). Also, how the first support 211 is coupled to, and located relative to, the fixation helix 110 and the first anti-reverse element 221 can be clearly seen. This additionally demonstrates that the presence of the first support 211 can not only ensure that the fixation helix 110, the first welding plate 131, the connecting member 120 and the first anti-reverse element 221 are coupled together securely, but can also effectively prevent the fixation helix 110 from excessive radial deformation.

### Example 3

In a third embodiment, there is provided an anti-reverse fixing structure 1. As mentioned earlier, the anti-reverse fixing structure 1 of this embodiment is as shown in Fig. 11. As can be seen from a comparison made between Fig. 11 and Figs. 1 and 2, the anti-reverse fixing structure 1 of this embodiment is substantially similar to the anti-reverse fixing structure 1 of the first embodiment and also includes a fixation substructure 100, an anti-reverse substructure 200 and a mounting substructure 300. The mounting substructure 300 in the anti-reverse fixing structure 1 of this embodiment also includes a mounting member 310 and a sealing disc 320. However, differing from the first embodiment, the fixation substructure 100 in the anti-reverse fixing structure 1 of this embodiment includes only a fixation helix 110 and a welding plate (for ease of distinction and description, the welding plate of the first embodiment has been termed as the "first welding plate 131", while that of this second embodiment is termed as a "second welding plate 132"), but not a connecting member 120, and the anti-reverse substructure 200 in the anti-reverse fixing structure 1 of this embodiment includes only an anti-reverse element (for ease of distinction and description, the anti-reverse element of the first embodiment has been termed as the "first anti-reverse element 221", while that of this second embodiment is termed as a "second anti-reverse element 222"), but not a support. Since the anti-reverse substructure 200 of this embodiment does not include a support, each of the second welding plate 132 and the mounting member 310 of this embodiment is correspondingly of a different structure from that of the first embodiment. To avoid repetition, the previously described features of the anti-reverse fixing structures 1 of the first and second embodiments that are shared with this embodiment, such as the fixation helix 110, the sealing disc 320 and the materials of the components are not repeated here.

Additionally, as noted above, a first anti-reverse feature 2221 in the anti-reverse fixing structure 1 of this embodiment is able to bend at its distal end so as to come into abutment, at least in part, with target organic tissue. Such bendability includes axially proximal bendability of the first anti-reverse feature 2221 at its distal end into abutment, at least in part, with the target organic tissue. It should be noted that, for any feature that is not detailed in this embodiment, reference is made to relevant accompanying drawings and the foregoing description of the first and second embodiments in connection with this feature for more details thereof that help appropriately understand it. Only differences from the first embodiment are described below.

Reference is now made to Figs. 11 and 12. Fig. 12 shows the fixation helix 110 and the second welding plate 132 of Fig. 11. As can be seen from Figs. 11 and 12, in some exemplary implementations, the fixation substructure 100 in the anti-reverse fixing structure 1 of this embodiment further includes the second welding plate 132, which is shaped like a ring, the distal end of the second welding plate 132 (i.e., the upper end of the second welding plate 132, as viewed in the orientation of Fig. 12) is provided with a second locating feature 1322 extending away from the anti-reverse substructure 200. A proximal end of the fixation helix 110 is brought into abutment with one side of the second locating feature 1322 so that the fixation helix 110 is coupled to the second welding plate 132 in a predetermined helical orientation. Through orienting the fixation helix 110 in the predetermined helical orientation, not only the fixation helix 110 can be more easily and quickly assembled with the second welding plate 132, but it can be ensured that the fixation helix 110 is fixedly coupled to the second welding plate 132 in the predetermined helical orientation, thereby providing for effectively prevention of unscrewing of the fixation helix 110.

In some exemplary implementations, the second welding plate 132 has a distal annular surface, which provides a third welding surface 1321. The fixation helix 110 defines a first welding surface 112, which is adapted to be welded to, and matches, the third welding surface 1321. With this arrangement, not only the presence of the third welding surface 1321 allows the fixation helix 110 to be more easily fixedly coupled to the second welding plate 132, but the annular second welding plate 132 can also provide good support for the fixation helix 110. Thus, the fixation helix 110 can be more securely coupled to the second welding plate 132 and exhibits high stiffness and withstanding capacity. Optionally, the second locating feature 1322 may be a bump-like protrusion. Specifically, in order to weld the fixation helix 110 to the second welding plate 132, the end of the fixation helix 110 with the first welding surface 112 may be first brought into abutment with a side of the second locating feature 1322, which faces toward the third welding surface 1321, and the first welding surface 112 may be then welded to the third welding surface 1321 in accordance with the predetermined helical orientation of the fixation helix 110. In this way, the first welding surface 112 can be attached to the third welding surface 1321 by welding so that the fixation helix 110 has the predetermined helical orientation.

Continued reference is made to Figs. 11 and 12. As can be seen from Figs. 11 and 12, in some exemplary implementations, when the fixation helix 110 is projected along its axis onto the third welding surface 1321, the projection is encompassed within the third welding surface 1321. With this arrangement, since the axial projection of the fixation helix 110 is encompassed within the third welding surface 1321, not only the fixation helix 110 can be more easily assembled with the second welding plate 132 by welding, enabling the second welding plate 132 to provide increased support for the fixation helix 110, but also the integral fabrication of the second anti-reverse element 222 can be facilitated, as described below. As can be more particularly seen from Figs. 11 and 12, the second welding plate 132 is stepped at its distal end so as to have a raised central portion and a lower peripheral portion, and the third welding surface 1321 is provided by an annular surface of the lower peripheral portion. With this arrangement, since the axial projection of the fixation helix 110 is encompassed within the third welding surface 1321 (i.e., the annular surface of the peripheral portion), the fixation helix 110 can be confined within a predetermined space by the raised central portion and the first anti-reverse feature 2221 of the second anti-reverse element 222, as described below. This ensures radial stability of the fixation helix 110 relative to the second welding plate 132, allowing the fixation helix 110 to deform along an axis of the anti-reverse fixing structure 1 in an even more reliable manner.

Optionally, the second welding plate 132 may be made of a biocompatible metallic material. Particular examples of the biocompatible metallic material for the second welding plate 132 may include, but are not limited to, stainless steel, nickel-titanium alloys, MP35N and 35NLT. For details of the advantages of such a biocompatible metallic material, reference is made to the above description in connection with the first welding plate 131, and further discussion thereof is omitted here.

Reference is now made to Fig. 11 and Fig. 13, Fig. 13 schematically illustrates the second anti-reverse element 222, the second welding plate 132 (not labeled in Fig. 13) and the fixation helix 110 of Fig. 11. In some exemplary implementations, the anti-reverse substructure 200 further includes a second anti-reverse element 222. The second anti-reverse element 222 includes the first anti-reverse feature 2221, a first anti-reverse base 2222 and at least one third anti-reverse feature 2223. Both the first and third anti-reverse features 2221, 2223 are arranged circumferentially around the first anti-reverse base 2222 at a distal end thereof so as to circumferentially extend in the same direction that is opposite to an unscrewing direction for the fixation helix 110. The first anti-reverse feature 2221 is located external to the third anti-reverse feature 2223. Part of a distal end face of the first anti-reverse base 2222 between the first and third anti-reverse features 2221, 2223 is coupled (optionally, fixedly) to a distal end of the second welding plate 132. A proximal end of the first anti-reverse base 2222 is coupled (optionally, fixedly) to a distal end of the mounting substructure 300. With this arrangement, the presence of the first anti-reverse base 2222 not only provides for the provision of the first and third anti-reverse features 2221, 2223, but also makes it easier for the second anti-reverse element 222 to be fixedly coupled to the second welding plate 132 and the mounting substructure 300 (more precisely, to the mounting member 310 therein, as described below). Compared with the first embodiment, the anti-reverse substructure 200 in the anti-reverse fixing structure 1 of this embodiment is simplified in structure so as to eliminate the need for assembly of the anti-reverse substructure 200 that is necessary for the first or second embodiment, enabling more efficient fabrication. Further, through designing the first and third anti-reverse features 2221, 2223 in such a manner that they are both arranged circumferentially around the first anti-reverse base 2222 at the distal end thereof so as to circumferentially extend in the same direction that is opposite to an unscrewing direction for the fixation helix 110, the first and third anti-reverse features 2221, 2223 are enabled to, when urged by the target organic tissue along the axis of the anti-reverse fixing structure 1, bend along the axis of the anti-reverse fixing structure 1 from its distal end to the proximal end so as to contact the target organic tissue over larger areas. As a result, an associated implantable medical device will encounter increased resistance when axially penetrating, as a whole, deeper into the target organic tissue (e.g., myocardial tissue), reducing the risk of the tissue being penetrated through. Furthermore, since part of the distal end face of the first anti-reverse base 2222 between the first and third anti-reverse features 2221, 2223 may be fixedly coupled to the distal end of the second welding plate 132, the first and third anti-reverse features 2221, 2223 can radially restrict the fixation helix 110, making the fixation helix 110 more stable.

Specifically, reference is made to Fig. 13. Similar to the first anti-reverse feature 2221 of the first embodiment, the first and third anti-reverse features 2221, 2223 of the second anti-reverse element 222 in the anti-reverse fixing structure 1 of this embodiment are also plate-like structures. The first anti-reverse feature 2221 also defines a first slanted surface 2221a, a second slanted surface 2221b and a third slanted surface 2221c. The first slanted surface 2221a forms a first angle with a cross-section of the anti-reverse fixing structure 1, which may be selected from the range of 30° to 60°. The second slanted surface 2221b forms a second angle with the cross-section of the anti-reverse fixing structure 1, which may be selected from the range of 60° to 90° (i.e., greater than the first angle formed between the first slanted surface 2221a and the cross-section of the anti-reverse fixing structure 1). That is, the second angle is greater than the first angle. With this design, because the first slanted surface 2221a is slanted in a direction opposite to the unscrewing direction for the fixation helix 110, and since the second angle is greater than the first angle, the anti-reverse fixing structure 1 will encounter even greater resistance when turned in the unscrewing direction than when turned in the opposite screwing direction. The third angle between the third slanted surface 2221c and the cross-section of the anti-reverse fixing structure 1 ranges from 30° to 60°. With this arrangement, the first anti-reverse feature 2221, when axially urged by the target organic tissue, will bend along the axis of the anti-reverse fixing structure 1 from the distal end to the proximal end of the anti-reverse fixing structure, resulting in a larger contact area between the first anti-reverse feature 2221 and the target organic tissue and hence increased resistance to deeper axial penetration of the target organic tissue (e.g., myocardial tissue) by an associated implantable medical device as a whole. Thus, the risk of the tissue being penetrated through can be lowered.

Similar to the first anti-reverse feature 2221, the third anti-reverse feature 2223 defines a first slanted surface 2223a and a second slanted surface 2223b. The first slanted surface 2223a forms a first angle optionally of 30° to 60° with the cross-section of the anti-reverse fixing structure 1, and the second slanted surface 2223b forms a second angle with the cross-section of the anti-reverse fixing structure 1, which may be generally selected from the range of 60° to 90° (greater than the angle formed between the first slanted surface 2223a and the cross-section of the anti-reverse fixing structure 1). That is, the second angle is greater than the first angle. With this design, because the first slanted surface 2223a is slanted in a direction opposite to the unscrewing direction for the fixation helix 110, and since the second angle is greater than the first angle, the anti-reverse fixing structure 1 will encounter still even greater resistance when turned in the unscrewing direction than when turned in the opposite screwing direction.

Optionally, the second anti-reverse element 222 may be made of a flexible biocompatible non-metallic material. Particular examples of the biocompatible non-metallic material for the second anti-reverse element 222 may include, but are not limited to, those capable of imparting a high degree of deformability, such as silicone, nylon, and fabrics made from animal hair and plant fibers. Further description in this regard is deemed unnecessary and omitted herein. The second anti-reverse element 222 is made of a flexible biocompatible non-metallic material and therefore easily deformable under the action of an external force applied thereto.

It should be noted that the present application is not limited to any particular number of first or third anti-reverse features 2221, 2223. The numbers respectively of first and third anti-reverse features 2221, 2223 may each be one, two or even more. However, there may optionally be two to eight first anti-reverse features 2221 and two to eight third anti-reverse features 2223.

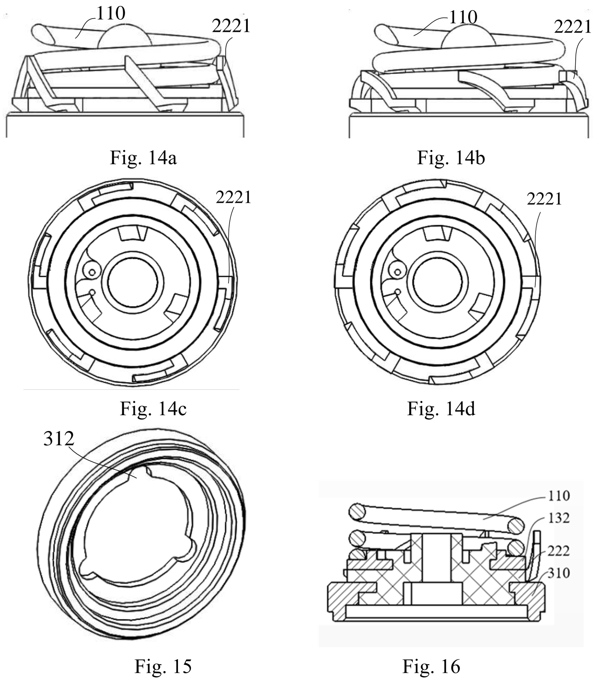

Specifically, reference is made to Figs. 14a to 14d. Fig. 14a shows an elevation view of the second anti-reverse element 222 of Fig. 11, which is being subject to a seventh external force acting along an axis of the anti-reverse fixing structure 1 from the distal end to the proximal end of the anti-reverse fixing structure 1. Fig. 14b shows an elevation view of the second anti-reverse element 222 of Fig. 11, which is being subject to an eighth external force acting along an axis of the anti-reverse fixing structure 1 from the distal end to the proximal end of the anti-reverse fixing structure 1. Fig. 14c schematically depicts the second anti-reverse element 222 of Fig. 14a, as viewed along the axis of the anti-reverse fixing structure 1 from the distal end to the proximal end of the anti-reverse fixing structure 1. Fig. 14d schematically depicts the second anti-reverse element 222 of Fig. 14b, as viewed along the axis of the anti-reverse fixing structure 1 from the distal end to the proximal end of the anti-reverse fixing structure 1. From Figs. 14a to 14d, bending of the first anti-reverse feature 2221 under the action of the different external forces (the seventh external force is smaller than the eighth external force) can be seen, which occurs along the axis of the anti-reverse fixing structure 1 toward its proximal end and leads to a reduced axial height of the first anti-reverse feature 2221 (and hence an increased contact area between the first anti-reverse feature 2221 and the target organic tissue). Also, how the fixation helix 110 and the second welding plate 132 are coupled to, and located relative to, the second anti-reverse element 222 can be clearly seen.

Continued reference is made to Fig. 11, 12, 13, 15 and 16. Fig. 15 schematically illustrates the mounting member 310 of Fig. 11. Fig. 16 shows an axial cross-sectional view of the fixation helix 110, the second welding plate 132, the second anti-reverse element 222 and the mounting member 310 of Fig. 11, which are integrally formed into one piece. As can be seen from Fig. 11, in some exemplary implementations, the first anti-reverse base 2222 defines a distal first annular step 22221 extending away from the mounting substructure 300 and having a distal outer diameter greater than its proximal outer diameter, along the axis of the anti-reverse fixing structure 1. The third anti-reverse feature 2223 is disposed at a distal end of the first annular step 22221, and the second welding plate 132 is disposed over a proximal end portion of the first annular step 22221. An inner side of the second welding plate 132 is complementary in shape to an outer side of the proximal end portion of the first annular step 22221. With this arrangement, the annular step provides for mutual nesting engagement of the second welding plate 132 with the first anti-reverse base 2222, which ensures that the second welding plate 132 is securely coupled to the second anti-reverse element 222 along the axis of the anti-reverse fixing structure 1.

Continued reference is made to Figs. 11 and 12. As can be seen from Figs. 11 and 12, in some exemplary implementations, at least one first limiting feature 1323 is formed on the inner side of the second welding plate 132, and the proximal end portion of the first annular step 22221 defines, on its outer side, a second limiting feature 22222 complementary in shape to the first limiting feature 1323. With this arrangement, the first limiting feature 1323 of the second welding plate 132 can engage the second limiting feature 22222 of the first anti-reverse base 2222, limiting the second welding plate 132 and the second anti-reverse element 222 from circumferential rotation relative to each other.

Optionally, in some exemplary implementations, a male/female fit may be formed between the first limiting feature 1323 and the second limiting feature 22222. In particular, the first limiting feature 1323 may be a through notch allowing passage therethrough along the axis of the anti-reverse fixing structure 1, and the second limiting feature 22222 may be a protuberance complementary in shape to the through notch. It should be noted that these implementations are optional implementations, and the present application is not limited thereto. In some other implementations, the first limiting feature 1323 may be provided instead as a protuberance, and the second limiting feature 22222 instead as a through notch. In yet some other implementations, the first and second limiting features 1323, 22222 may be provided as intermeshing teeth. While not described herein, further implementations are also possible.

Optionally, two to eight first limiting features 1323 and two to eight second limiting features 22222 may be provided. With this arrangement, the two to eight first limiting features 1323 and the two to eight second limiting features 22222 can ensure that the first and second limiting features 1323, 22222 are well circumferentially equidistantly spaced at appropriate intervals without complicating the fabrication of the second welding plate 132 and the second anti-reverse element 222 too much. Thus, not only the second welding plate 132 can be limited from circumferential rotation, but the second welding plate 132 and the second anti-reverse element 222 can be effectively restrained from moving relative to each other in a radial direction of the anti-reverse fixing structure 1, thereby enabling more secure engagement of the second welding plate 132 and the second anti-reverse element 222 in radial directions of the anti-reverse fixing structure 1.

In some exemplary implementations, with continued reference to Fig. 11, 15 and 16, the first anti-reverse base 2222 defines a proximal second annular step 22223 (i.e., at the right-hand side of the first anti-reverse base 2222, as viewed in the orientation of Fig. 11) extending toward the mounting substructure 300 and having a proximal outer diameter greater than its distal outer diameter, along the axis of the anti-reverse fixing structure 1. The mounting member 310 in the mounting substructure 300 is disposed over an outer side of a distal end portion of the second annular step 22223, and an inner side of the mounting member 310 is complementary in shape to the outer side of the distal end portion of the second annular step 22223. With this arrangement, the annular step provides for mutual nesting engagement of the second anti-reverse base 2232 in the second anti-reverse element 222 with the mounting member 310, which ensures that the second anti-reverse element 222 is securely coupled to the mounting member 310 along the axis of the anti-reverse fixing structure 1.

In some exemplary implementations, with continued reference to Fig. 15, the mounting member 310 is in the shape of a ring, with at least one third limiting feature 312 being formed on its inner side. The distal end portion of the second annular step 22223 defines, on its outer side, a fourth limiting feature 22224 complementary in shape to the third limiting feature 312. With this arrangement, the third limiting feature 312 of the mounting member 310 can engage the fourth limiting feature 22224 of the first anti-reverse base 2222, limiting the mounting member 310 and the second anti-reverse element 222 from circumferential rotation relative to each other.

Optionally, a male/female fit may be formed between the third limiting feature 312 and the fourth limiting feature 22224. In particular, the third limiting feature 312 may be a through notch allowing passage therethrough along the axis of the anti-reverse fixing structure 1, and the fourth limiting feature 22224 may be a protuberance complementary in shape to the through notch. It should be noted that these implementations are optional implementations, and the present application is not limited thereto. In some other implementations, the third limiting feature 312 may be provided instead as a protuberance, and the fourth limiting feature 22224 instead as a through notch. In yet some other implementations, the third and fourth limiting features 312, 22224 may be provided as intermeshing teeth. While not described herein, further implementations are also possible.

Optionally, two to eight third limiting features 312 and two to eight fourth limiting features 22224 may be provided. With this arrangement, the two to eight third limiting features 312 and the two to eight fourth limiting features 22224 can ensure that the third and fourth limiting features 312, 22224 are well circumferentially equidistantly spaced at appropriate intervals without complicating the fabrication of the second welding plate 132 and the second anti-reverse element 222 too much. Thus, not only the second anti-reverse element 222 can be limited from circumferential rotation, but the second anti-reverse element 222 and the mounting member 310 can be effectively restrained from moving relative to each other in a radial direction of the anti-reverse fixing structure 1, thereby enabling more secure engagement of the second anti-reverse element 222 and the mounting member 310 in radial directions of the anti-reverse fixing structure 1.

### Example 4

In a fourth embodiment, there is provided an anti-reverse fixing structure 1. As mentioned earlier, the anti-reverse fixing structure 1 of this embodiment is as shown in Fig. 17. As can be seen from a comparison made between Fig. 17 and Figs. 1 and 2, the anti-reverse fixing structure 1 of this embodiment is substantially similar to the anti-reverse fixing structure 1 of the first embodiment and also includes a fixation substructure 100, an anti-reverse substructure 200 and a mounting substructure 300. The mounting substructure 300 in the anti-reverse fixing structure 1 of this embodiment also includes a mounting member 310 and a sealing disc 320. However, differing from the first embodiment, the fixation substructure 100 in the anti-reverse fixing structure 1 of this embodiment includes only a fixation helix 110, but not a welding plate or a connecting member 120. In other words, it neither includes the connecting member 120 and the first welding plate 131 of the first embodiment, nor includes the second welding plate 132 of the third embodiment. Moreover, differing from the anti-reverse substructure 200 in the anti-reverse fixing structure 1 of the third embodiment that includes only the anti-reverse element (for ease of distinction and description, the anti-reverse element of the third embodiment has been termed as the "second anti-reverse element 222"), the anti-reverse substructure 200 in the anti-reverse fixing structure 1 of the this embodiment includes an anti-reverse element (for ease of distinction and description, the anti-reverse element of the first embodiment has been termed as the "first anti-reverse element 221", while that of this embodiment is termed as a "third anti-reverse element 223") and a support (for ease of distinction and description, the support of the first embodiment has been termed as the "first support 211", while that of this embodiment is termed as a "second support 212"). However, differing from the first embodiment, in which the first support 211 is disposed closer to the fixation helix 110 and the first anti-reverse element 221 to the fixation substructure 100, in this embodiment, the third anti-reverse element 223 is disposed closer to the fixation helix 110 and the second support 212 to the fixation substructure 100. To avoid repetition, only differences from the first embodiment are described below, and for any feature that is not detailed in this embodiment, reference is made to relevant accompanying drawings and the foregoing description of the first, second and/or third embodiments in connection with this feature for more details thereof that help appropriately understand it.

Specifically, reference is made to Figs. 17, 18 and 19. Fig. 18 is a schematic illustration of the fixation helix 110 and the third anti-reverse element 223 of Fig. 17. Fig. 19 is a schematic illustration of the second support 212 of Fig. 17. As can be seen from Figs. 17, 18 and 19, in some exemplary implementations, the anti-reverse substructure 200 in the anti-reverse fixing structure 1 of this embodiment includes the third anti-reverse element 223 and the second support 212. The third anti-reverse element 223 includes a first anti-reverse feature 2231 and a second anti-reverse base 2232. The first anti-reverse feature 2231 is provided on the second anti-reverse base 2232 (if multiple first anti-reverse features 2231 are present, they are spaced circumferentially around the second anti-reverse base 2232). A distal end face of the second anti-reverse base 2232 is coupled (optionally, fixedly) to a proximal end of the fixation helix 110, and a proximal end of the second anti-reverse base 2232 is coupled (optionally, fixedly) to a distal end of the second support 212. A proximal end of the second support 212 is coupled (optionally, fixedly) to a distal end of the mounting substructure 300 (optionally, to the mounting member 310). Optionally, the second anti-reverse base 2232 may be integrally injection molded with a third anti-reverse base 2121 described below and the mounting member 310. With this arrangement, since the proximal end of the fixation helix 110 is fixedly coupled to the distal end face of the second anti-reverse base 2232, the second anti-reverse base 2232 can provide support for the fixation helix 110. Moreover, since the proximal end of the second anti-reverse base 2232 is fixedly coupled to the distal end of the second support 212, the second support 212 can provide sufficient support for the second anti-reverse base 2232. Additionally, this arrangement enables the first anti-reverse feature 2231, when stressed at a distal end thereof by an external force acting along an axis of the anti-reverse fixing structure 1 from the distal end to the proximal end of the anti-reverse fixing structure, to bend at the distal end along the axis of the anti-reverse fixing structure 1 from the distal end to the proximal end of the anti-reverse fixing structure so as to come into abutment, at least in part, with target organic tissue. Further, the above discussed design of the second anti-reverse base 2232 facilitates the integral formation of the third anti-reverse element 223.

Referring to Fig. 17, in some exemplary implementations, the second anti-reverse base 2232 defines a distal third annular step 22321 extending away from the mounting substructure 300 and having an outer diameter smaller than an outer diameter of the rest of the second anti-reverse base 2232, along the axis of the anti-reverse fixing structure 1. The first anti-reverse feature 2231 is disposed on a circumferential side wall of the second anti-reverse base 2232, and the fixation helix 110 is coupled (optionally, fixedly) at its proximal end to the distal end face of the second anti-reverse base 2232 while being disposed over the third annular step 22321. With this arrangement, since the fixation helix 110 is disposed over the third annular step 22321 and coupled to the distal end face of the second anti-reverse base 2232, it can provide sufficient support for the fixation helix 110. Coupled with the first anti-reverse feature 2231 arranged on the circumferential side wall of the second anti-reverse base 2232, this can confine the fixation helix 110 within a predetermined space, facilitating radially locating of the fixation helix 110 and hence ensuring radial stability of the fixation helix 110 relative to the third anti-reverse element 223.

With continued reference to Figs. 17, 18 and 19, in some exemplary implementations, an inner side of the third annular step 22321 is complementary in shape to a distal end portion of the second support 212. With this arrangement, since the inner side of the third annular step 22321 is complementary in shape to the distal end portion of the second support 212, the third annular step 22321 can be more securely coupled to the second support 212.

In some exemplary implementations, as shown in Fig. 18, the second anti-reverse base 2232 is provided on its proximal end face with a third locating feature 22322 extending toward the fixation substructure 100. The proximal end of the fixation helix 110 is brought into abutment with one side of the third locating feature 22322 so that the fixation helix 110 is coupled to the second anti-reverse base 2232 in a predetermined helical orientation. Optionally, the third locating feature 22322 may be a bump-like protrusion defining an axial thickness, which matches a thickness of the abutted end portion of the fixation helix 110. Through orienting the fixation helix 110 in the predetermined helical orientation with the aid of the third locating feature 22322, not only easier and quicker assembly of the fixation helix 110 with the third anti-reverse element 223 (simplified into assembly of the fixation helix 110 with the second anti-reverse base 2232) can be achieved, but it can be ensured that the fixation helix 110 is fixedly coupled to the second welding plate 132 in the predetermined helical orientation, thereby providing for effectively prevention of unscrewing of the fixation helix 110.

In some exemplary implementations, the proximal end face of the second anti-reverse base 2232 defines a fourth welding surface 22323 contiguous with the third locating feature 22322, and the fixation helix 110 defines a first welding surface 112 for being welded to the fourth welding surface 22323. The first anti-reverse feature 2231 is located external to the fourth welding surface 22323. With this arrangement, not only the presence of the fourth welding surface 22323 allows the fixation helix 110 to be fixedly coupled to the third anti-reverse element 223 (simply by assembling the fixation helix 110 with the second anti-reverse base 2232), but the annular fourth welding surface 22323 (provided by the third annular step 22321) can provide good support for the fixation helix 110. Thus, the fixation helix 110 can be more securely coupled to the third anti-reverse element 223 and exhibits high stiffness and withstanding capacity. Optionally, one radial side of the third locating feature 22322 is brought into abutment with the fourth welding surface 22323. Specifically, in order to welding the fixation helix 110 to the third anti-reverse element 223, the end of the fixation helix 110 with the first welding surface 112 may be first brought into abutment with a side of the third locating feature 22322 facing toward the fourth welding surface 22323, and the first welding surface 112 may be then welded to the fourth welding surface 22323 so that the fixation helix 110 has the predetermined helical orientation. In this way, the first welding surface 112 can be attached to the fourth welding surface 22323 by welding so that the fixation helix 110 is oriented in the predetermined helical orientation.

With continued reference to Figs. 17 and 18, in some exemplary implementations, when the fixation helix 110 is projected along its axis onto the fourth welding surface 22323, the projection is encompassed within the fourth welding surface 22323. With this arrangement, since the axial projection of the fixation helix 110 is encompassed within the fourth welding surface 22323, the fixation helix 110 can be more easily assembled with the third anti-reverse element 223.

In particular, the third anti-reverse element 223 may be made of a flexible biocompatible metallic material. With this arrangement, on the one hand, the flexible biocompatible metallic material (optionally selected as a biocompatible metallic material capable of imparting a high degree of deformability) enables the third anti-reverse element 223 to be compatible with target organic tissue, without causing any immune reaction or rejection, thereby ensuring good safety after the anti-reverse fixing structure 1 is implanted to the target organic tissue. On the other hand, the metallic material can provide sufficient support for the fixation helix 110. Particular examples of the biocompatible metallic material may include, but are not limited to, stainless steel, nickel-titanium alloys, MP35N and 35NLT.

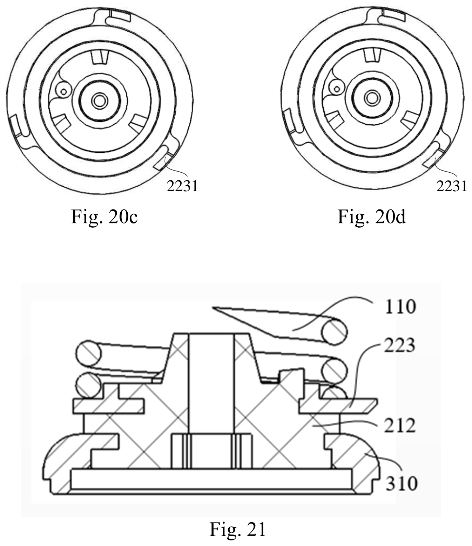

Specifically, reference is made to Figs. 20a to 20d. Fig. 20a shows an elevation view of the third anti-reverse element 223 of Fig. 17, which is being subject to a ninth external force acting along the axis of the anti-reverse fixing structure 1 from the distal end to the proximal end of the anti-reverse fixing structure 1. Fig. 20b shows an elevation view of the third anti-reverse element 223 of Fig. 17, which is being subject to a tenth external force acting along the axis of the anti-reverse fixing structure 1 from the distal end to the proximal end of the anti-reverse fixing structure 1. Fig. 20c schematically depicts the third anti-reverse element 223 of Fig. 20a, as viewed along the axis of the anti-reverse fixing structure 1 from the distal end to the proximal end of the anti-reverse fixing structure 1. Fig. 20d schematically depicts the third anti-reverse element 223 of Fig. 20b, as viewed along the axis of the anti-reverse fixing structure 1 from the distal end to the proximal end of the anti-reverse fixing structure 1. From Figs. 20a to 20d, bending of the first anti-reverse feature 2231 under the action of the different external forces (the ninth external force is smaller than the tenth external force) can be seen, which occurs along the axis of the anti-reverse fixing structure 1 toward its proximal end and leads to a reduced axial height of the first anti-reverse feature 2231 (and hence an increased contact area between the first anti-reverse feature 2231 and the target organic tissue). Also, how the third anti-reverse element 223 is coupled to, and located relative to, the fixation helix 110, the second support 212 and the mounting member 310 can be clearly seen. This additionally demonstrates that the presence of the second support 212 can ensure that the fixation helix 110, the third anti-reverse element 223 and the mounting member 310 are coupled together securely and that the second support 212 can work with the third anti-reverse element 223 and the mounting member 310 to effectively prevent the fixation helix 110 from excessive radial deformation.

Continued reference is made to Figs. 17, 19 and 21. Fig. 21 shows an axial cross-sectional view of the fixation helix 110, the third anti-reverse element 223, the second support 212 and the mounting member 310 of Fig. 17, which are integrally formed into one piece. As can be seen from Figs. 17, 19 and 21, the second support 212 includes a third anti-reverse base 2121. In some implementations, the third anti-reverse base 2121 defines a distal fourth annular step 21211 extending toward the fixation substructure 100 and having a distal outer diameter greater than its proximal outer diameter, along the axis of the anti-reverse fixing structure 1. The second anti-reverse base 2232 is disposed over an outer side of the fourth annular step 21211 at a proximal end thereof. Through disposing the second anti-reverse base 2232 over the outer side of the fourth annular step 21211 at its proximal end, not only sufficient support can be provided for the third anti-reverse element 223 and hence for the fixation helix 110, but it can be ensured that third anti-reverse element 223 is securely coupled to the second support 212 along the axis of the anti-reverse fixing structure 1.

Additionally, in some exemplary implementations, the third anti-reverse base 2121 defines a proximal fifth annular step 21212 extending toward the mounting substructure 300, the fifth annular step 21212 has a distal outer diameter smaller than its proximal outer diameter along the axis of the anti-reverse fixing structure 1. The mounting substructure 300 includes the mounting member 310. The mounting member 310 is disposed over an outer side of the fifth annular step 21212 at a distal end thereof, and an inner side of the mounting member 310 is complementary in shape to the outer side of the fifth annular step 21212 at its distal end. Through disposing the second anti-reverse base 2232 over the outer side of the fourth annular step 21211 at its proximal end, not only sufficient support can be provided for the third anti-reverse element 223 and hence for the fixation helix 110, but it can be ensured that the third anti-reverse element 223 is securely coupled to the second support 212 along the axis of the anti-reverse fixing structure 1.

In particular, the second support 212 may be made of a biocompatible non-metallic material, such as PP, silicone, PE, PU, nylon, PU, PI, PEEK, PC or the like.

In some optional implementations, the third annular step 22321 delimits a circular inner bore, which extends through the second anti-reverse base 2232. Moreover, at least one fifth limiting feature 22324 is provided on the inner side of the third annular step 22321, and a sixth limiting feature 21214 complementary in shape to the fifth limiting feature 22324 is provided on the outer side of the fourth annular step 21211 at its proximal end. With this arrangement, the fifth limiting feature 22324 and the sixth limiting feature 21214 can cooperate to limit the third anti-reverse element 223 and the second support 212 from circumferential rotation relative to each other.

Optionally, a male/female fit may be formed between the fifth limiting feature 22324 and the sixth limiting feature 21214. Optionally, two to eight fifth limiting features 22324 and two to eight sixth limiting features 21214 may be provided. Since the quantities of the fifth and sixth limiting features 22324, 21214 and their mating and engagement are similar to those of the above discussed first and second limiting features 1323, 22222, in order to avoid repetition, further description of their advantages is omitted here, and reference is made to the above description in connection with the first and second limiting features 1323, 22222 for more details that help appropriately understand such advantages.

Further, in some exemplary implementations, the fifth annular step 21212 is provided on its outer side at the distal end thereof with at least one seventh limiting feature 21215, and the mounting member 310 is shaped like a ring (reference is made to the above description of the third embodiment in connection with Fig. 15). On the inner side of the mounting member 310, at least one third limiting feature 312 is formed, which is complementary in shape to the seventh limiting feature 21215. With this arrangement, the seventh limiting feature 21215 and the third limiting feature 312 can work together to limit the second support 212 and the mounting member 310 from circumferential rotation relative to each other.

Optionally, a male/female fit may be formed between the seventh limiting feature 21215 and the third limiting feature 312. Optionally, two to eight seventh limiting features 21215 and two to eight third limiting features 312 may be provided. Since the quantities of the seventh and third limiting features 21215, 312 and their mating and engagement are similar to those of the above discussed first and second limiting features 1323, 22222, in order to avoid repetition, further description of their advantages is omitted here, and reference is made to the above description in connection with the first and second limiting features 1323, 22222 for more details that help appropriately understand such advantages.

In one optional implementation, with continued reference to Fig. 17, a support base 21213 is disposed between the fourth annular step 21211 and the fifth annular step 21212. The support base 21213 has an outer diameter matching the outer diameter of the second anti-reverse base 2232. Through providing the support base 21213 of the second support 212 between the fourth annular step 21211 and the fifth annular step 21212, on the one hand, the second support 212 can be more easily fixedly coupled to the third anti-reverse element 223, and more easily coupled to the mounting member 310. On the other hand, since the outer diameter of the support base 21213 matches the outer diameter of the second anti-reverse base 2232, sufficient support can be provided for the third anti-reverse element 223 without causing a radial expansion, thereby allowing the first anti-reverse feature 2231, when stressed at the distal end thereof by an external force acting along the axis of the anti-reverse fixing structure 1 from the distal end to the proximal end of the anti-reverse fixing structure, to bend at the distal end along the axis of the anti-reverse fixing structure 1 from the distal end to the proximal end of the anti-reverse fixing structure so as to come into abutment, at least in part, with the target organic tissue.

Reference is now made to Figs. 17 and 19. As can be seen from Fig. 19, in some exemplary implementations, the second support 212 includes at least one fourth anti-reverse feature 2122, which is disposed on a circumference of a distal end face of the fourth annular step 21211 and extends in a direction opposite to an unscrewing direction for the fixation helix 110. The fourth anti-reverse feature 2122 of this embodiment functions in a similar way to the third anti-reverse feature 2223 of the preceding third embodiment. Accordingly, further description thereof is omitted here, and reference is made to the above description in connection with the third anti-reverse feature 2223 for more details that help appropriately understand the function.

Optionally, the fixation helix 110 may be disposed in a gap between the first anti-reverse feature 2231 and the fourth anti-reverse feature 2122, with its axis being oriented parallel to the axis of the anti-reverse fixing structure 1. The relative positioning of the first anti-reverse feature 2231, the fixation helix 110 and the fourth anti-reverse feature 2122 in this embodiment is similar to that of the first anti-reverse feature 2221, the fixation helix 110 and the third anti-reverse feature 2223 of the preceding third embodiment. Reference is made to the above relevant description for more details that help appropriately understand such positioning.

In one exemplary implementation, the fourth anti-reverse feature 2122 defines a fifth slanted surface 2122a and a sixth slanted surface 2122b contiguous with the fifth slanted surface 2122a. Moreover, the sixth slanted surface 2122b is slanted in a direction opposite to the unscrewing direction for the fixation helix 110. The fifth slanted surface 2122a forms a fourth angle with a cross-section of the anti-reverse fixing structure 1, and the sixth slanted surface 2122b forms a fifth angle with the cross-section of the anti-reverse fixing structure 1. Both the fourth and fifth angles are non-zero, and the fourth angle is smaller than the fifth angle. The fourth anti-reverse feature 2122 of this embodiment functions in a similar way to the third anti-reverse feature 2223 of the preceding third embodiment. Reference is made to the above description in connection with the third anti-reverse feature 2223 for more details that help appropriately understand the function.

### Example 5

In a fifth embodiment, there is provided an implantable medical device, as particularly shown in Fig. 1, in conjunction with Figs. 2, 7, 11 and 17. The implantable medical device includes a device body (not labeled, including an electrical component 2, a power supply component 3, a housing 4 and a distal docking member 5, as shown in Fig. 1), an electrode 400, a feedthrough 500 and the anti-reverse fixing structure 1 of any of the foregoing embodiments. The electrode 400 is coupled (optionally, fixedly) to a distal end of the anti-reverse fixing structure 1, and a proximal end of the anti-reverse fixing structure 1 is coupled (optionally, fixedly) to a distal end of the device body. At least one feedthrough wire 600 of the feedthrough 500 is passed through the anti-reverse fixing structure 1 and electrically connected to the electrode 400.

Since the implantable medical device of this embodiment shares the same inventive concept as the anti-reverse fixing structures 1 of the foregoing embodiments, it has at least all the advantages of the anti-reverse fixing structures 1. For brevity, these advantages are not repeated here and reference is made to the above description in connection with the anti-reverse fixing structures 1 for more details.

In one exemplary implementation, the implantable medical device further includes a drug pill 700 disposed between the electrode 400 and the anti-reverse fixing structure 1. With this arrangement, the drug can be more easily delivered to target tissue.

It should be noted that the present application is not limited to any particular shape of the electrode 400 or the drug pill 700. For example, a tip portion of the electrode 400 may have various shapes, such as flat, oval, spherical, pointed, parabolic, etc. In addition, a proximal end of the electrode 400 may be spaced from the distal end of the fixation helix 110 at a distance, which can be determined according to test results generally in the range of 0 mm to 1.8 mm. Further, a groove feature (not labeled) for receiving the drug pill 700 may be formed at a distal end of the electrode 400.

Without limitation, the drug pill 700 may be made of a mixture of a silicone resin (e.g., a LOCTITE^{®} silicone adhesive) and a steroid active ingredient (e.g., dexamethasone acetate).

Reference throughout this specification to "one embodiment", "some embodiments", "an example", "a specific example" "some examples" or the like means that a particular feature, structure, material, or characteristic described in connection with the embodiments or examples is included in at least one embodiment or example of the present application. Thus, the appearances of those phrases in various places throughout this specification are not necessarily referring to the same embodiment or example of the application. Additionally, the particular features, structures, materials, or characteristics may be combined in any suitable manner in any one or more embodiments. Further, should there be no contradiction, one of ordinary skill in the art can combine the various embodiments, examples and features thereof described herein in any combination.

The present application offers the following benefits over the prior art:
It provides an anti-reverse fixing structure including: a fixation substructure for fixing an implantable medical device to target tissue; an anti-reverse substructure; and a mounting substructure for being coupled to a distal end of the implantable medical device. With this arrangement, not only the anti-reverse fixing structure can easily fix the implantable medical device to target organic tissue, but a modular design is provided, which makes the anti-reverse fixing structure simple in structure and easy to fabricate, assemble and maintain, resulting in manpower, material and time savings. Additionally, in the anti-reverse fixing structure, the anti-reverse substructure includes at least one first anti-reverse feature, which, when stressed at its distal end by an external force acting along an axis of the anti-reverse fixing structure from a distal end to a proximal end thereof, will bend at its distal end outwardly away from the axis of the anti-reverse fixing structure so as to come into abutment, at least in part, with the target organic tissue. With this arrangement, the anti-reverse fixing structure can not only effectively prevent unscrewing of the implantable medical device (e.g., a leadless pacemaker) away from the target organic tissue (e.g., myocardial tissue), but can also prevent the implantable medical device (e.g., a leadless pacemaker) from incrementally moving deeper into the target organic tissue (e.g., myocardial tissue) and finally penetrating through the tissue. Thus, higher reliability and increased safety can be obtained after the implantable medical device is implanted to the target organic tissue.

It also provides an implantable medical device, which is of the same inventive concept as the anti-reverse fixing structure and thereby has at least all the advantages of the anti-reverse fixing structure. For brevity, these advantages are not repeated here, and reference is made to the above description in connection with the anti-reverse fixing structure for more details.

Various anti-reverse fixing structure and implantable medical device configurations disclosed herein have been described in detail above with respect to the foregoing embodiments. Of course, the above description is merely that of a few preferred modes of carrying out the invention and is in no way intended to limit the scope thereof. Possible configurations of the present invention include, but are not limited to, those described in the foregoing embodiments, and those skilled in the art can obtain in light of the above teachings. Accordingly, any and all variations and modification made by those of ordinary skill in the art to which the present invention pertains in light of the above teachings are intended to fall within the scope thereof as defined by the appended claims.

## Claims

1. An anti-reverse fixing structure, **characterized by** being used for an implantable medical device; the anti-reverse fixing structure comprising: a fixation substructure for fixing the implantable medical device to a target tissue; an anti-reverse substructure; and a mounting substructure for being connected to a distal end of the implantable medical device,
the fixation substructure connected to the mounting substructure through the anti-reverse substructure, and
the anti-reverse substructure comprising at least one first anti-reverse feature, when a distal end of the first anti-reverse feature is subjected to an external force acting along an axis of the anti-reverse fixing structure from a distal end to a proximal end of the anti-reverse fixing structure, the distal end of the first anti-reverse feature is capable of deforming.

2. The anti-reverse fixing structure according to claim 1, wherein the anti-reverse substructure comprises a plurality of first anti-reverse features, and the plurality of first anti-reverse features arranged around an axis of the anti-reverse fixing structure.

3. The anti-reverse fixing structure according to claim 1, wherein the first anti-reverse feature is made of a flexible biocompatible material and the deformation that the first anti-reverse feature is capable of undergoing causes a height of the first anti-reverse feature along the axis to decrease and/or a projection area of the first anti-reverse feature along the axis to increase.

4. The anti-reverse fixing structure according to claim 2, wherein the fixation substructure comprises a fixation helix for connecting to a target organic tissue, the fixation helix defining an axis in coincidence with the axis of the anti-reverse fixing structure, a proximal end of the fixation helix connected to a distal end of the anti-reverse substructure, and wherein the axis of the anti-reverse fixing structure is defined by a line connecting the proximal end to the distal end of the anti-reverse fixing structure.

5. The anti-reverse fixing structure according to claim 4, wherein the fixation substructure further comprises a first welding plate, a distal end of the first welding plate connected to the proximal end of the fixation helix, and a proximal end of the first welding plate connected to the distal end of the anti-reverse substructure.

6. The anti-reverse fixing structure according to claim 5, wherein the first welding plate is made of a biocompatible metallic material.

7. The anti-reverse fixing structure according to claim 5, wherein a first welding surface is provided at the proximal end of the fixation helix, a second welding surface is provided at the distal end of the first welding plate, the first welding surface connected to the second welding surface.

8. The anti-reverse fixing structure according to claim 7, wherein the first welding plate further comprises a first locating feature for locating a helical orientation of the fixation helix, the first locating feature configured so that the first welding surface of the fixation helix is connected to the second welding surface by welding according to a predetermined helical orientation.

9. The anti-reverse fixing structure according to claim 5, wherein the first welding plate further comprises a welding plate mounting feature disposed at the proximal end of the first welding plate, and the welding plate mounting feature complementary in shape to the distal end of the anti-reverse substructure and connected to the distal end of the anti-reverse substructure.

10. The anti-reverse fixing structure according to claim 4, wherein the fixation substructure further comprises a connecting member, the connecting member at least partially disposed within the fixation helix along the axis of the anti-reverse fixing structure, a distal end of the connecting member configured to secure an electrode of the implantable medical device, and a proximal end of the connecting member connected to the distal end of the anti-reverse substructure.

11. The anti-reverse fixing structure according to claim 10, wherein the anti-reverse substructure comprises a first support and a first anti-reverse element circumferentially engaged with each other, a distal end of the first support connected to a portion of the fixation helix and the proximal end of the connecting member, a proximal end of the first support connected to a distal end of the mounting substructure, and
the first anti-reverse element comprising the first anti-reverse features and an anti-reverse element mounting feature, the first anti-reverse features provided on the anti-reverse element mounting feature.

12. The anti-reverse fixing structure according to claim 11, wherein distal ends of the first anti-reverse features are bendable and at least partially abutting against the target organic tissue, the bendability comprising radially outward bendability of the distal ends of the first anti-reverse features and at least partially abutting against the target organic tissue.

13. The anti-reverse fixing structure according to claim 12, wherein the first support defines side grooves and a bottom groove, wherein the first anti-reverse features are in positional correspondence with and engaged in the respective side grooves, and wherein the anti-reverse element mounting feature is located in the bottom groove and sandwiched between the first support and the mounting substructure.

14. The anti-reverse fixing structure according to claim 13, wherein the first anti-reverse features extend in a direction pointing from a proximal end to a distal end of the first anti-reverse element, and wherein the anti-reverse element mounting feature is shaped like a ring and fitted in the bottom groove of the first support.

15. The anti-reverse fixing structure according to claim 14, wherein engagement blocks are provided at intervals on an inner surface of the anti-reverse element mounting feature, the engagement blocks extend inwards along a direction perpendicular to the axis of the anti-reverse fixing structure, and
in the bottom groove, mating engagement grooves are formed at locations corresponding to the respective engagement blocks, and the engagement blocks extend into the respective engagement grooves.

16. The anti-reverse fixing structure according to claim 12, wherein a groove feature is formed at the distal end of the first support, wherein a helix mounting feature is formed in an inner wall surface of the groove feature, and wherein a portion of the fixation helix is retained in the helix mounting feature.

17. The anti-reverse fixing structure according to claim 16, wherein the helix mounting feature is a helical groove, the portion of the fixation helix retained in the helical groove complementary in shape to the helical groove.

18. The anti-reverse fixing structure according to claim 17, wherein the first support further comprises at least one second anti-reverse feature, which is provided in the inner wall surface of the groove feature and brought into communication with the helix mounting feature, the second anti-reverse feature implemented as a recess structure, an opening direction of the recess structure is the same as an opening direction of the helical groove, and a distal end of the recess structure is also in an open state.

19. The anti-reverse fixing structure according to claim 16, wherein the fixation helix is a variable pitch structure.

20. The anti-reverse fixing structure according to claim 16, wherein the first support further comprises an axial central feature provided at a bottom of the groove feature, the axial central feature complementary in shape to the proximal end of the connecting member and a welding plate mounting feature provided at a proximal end of a first welding plate in the fixation substructure, and wherein the connecting member and the first welding plate are connected to the first support by the axial central feature.

21. The anti-reverse fixing structure according to claim 12, wherein: each of the first anti-reverse features is a plate-like structure, which defines a first slanted surface forming a first angle with a cross-section of the anti-reverse fixing structure, a second slanted surface forming a second angle with the cross-section of the anti-reverse fixing structure and a third slanted surface forming a third angle with the cross-section of the anti-reverse fixing structure,
the first slanted surface is located at an end of the plate-like structure away from the anti-reverse element mounting feature and slanted in a direction opposite to an unscrewing direction for the fixation helix; the second slanted surface and the third slanted surface both extend from a proximal end to a distal end of the plate-like structure; the third slanted surface is contiguous with the first slanted surface and the second slanted surface; the first angle, the second angle, and the third angle are all non-zero; and the first angle is smaller than the second angle, and the third angle is smaller than 90°.

22. The anti-reverse fixing structure according to claim 21, wherein each of the first anti-reverse features further defines a fourth slanted surface, the fourth slanted surface contiguous with the first, second and third slanted surfaces, the fourth slanted surface defining the distal end of the plate-like structure together with the first slanted surface; and wherein along a circumferential direction of the anti-reverse element mounting feature, the first slanted surface extends a greater length than the fourth slanted surface.

23. The anti-reverse fixing structure according to claim 11, wherein the mounting substructure comprises a mounting member with a passage hole, and wherein the proximal end of the first support is inserted into the passage hole and connected to the mounting member.

24. The anti-reverse fixing structure according to claim 4, wherein the distal ends of the first anti-reverse features are bendable and at least partially abutting against the target organic tissue, the bendability comprising the distal ends of the first anti-reverse features are bendable axially toward the proximal end and at least partially abutting against the target organic tissue.

25. The anti-reverse fixing structure according to claim 24, wherein the fixation substructure further comprises an annular second welding plate, a distal end of the second welding plate provided with a second locating feature extending away from the anti-reverse substructure, and wherein the proximal end of the fixation helix is brought into abutment with one side of the second locating feature and causes the fixation helix to be connected to the second welding plate according to a predetermined helical orientation.

26. The anti-reverse fixing structure according to claim 25, wherein a third welding surface is provided on an annular surface at the distal end of the second welding plate, and wherein the fixation helix defines a first welding surface for being welded to the third welding surface.

27. The anti-reverse fixing structure according to claim 26, wherein a projection of the fixation helix along its axis onto the third welding surface is encompassed within the third welding surface.

28. The anti-reverse fixing structure according to claim 25, wherein the anti-reverse substructure further comprises a second anti-reverse element, the second anti-reverse element comprising the first anti-reverse features, a first anti-reverse base and at least one third anti-reverse feature, the first and third anti-reverse features both circumferentially arranged on a distal end of the first anti-reverse base and extending circumferentially in the same direction that is opposite to an unscrewing direction for the fixation helix, the first anti-reverse features located on an outer side of the third anti-reverse feature, a distal end face portion of the first anti-reverse base between the first and third anti-reverse features connected to a distal end of the second welding plate, and a proximal end of the first anti-reverse base connected to a distal end of the mounting substructure.

29. The anti-reverse fixing structure according to claim 28, wherein the distal end of the first anti-reverse base defines a first annular step extending away from the mounting substructure, along the axis of the anti-reverse fixing structure, an outer diameter of a distal end of the first annular step greater than an outer diameter of its proximal end, wherein the third anti-reverse feature is disposed at the distal end of the first annular step, and
the second welding plate is disposed over an outer side of the proximal end of the first annular step, an inner side of the second welding plate being complementary in shape to the outer side of the proximal end of the first annular step.

30. The anti-reverse fixing structure according to claim 29, wherein at least one first limiting feature is formed on the inner side of the second welding plate, and wherein an outer side of the proximal end of the first annular step defines a second limiting feature complementary in shape to the first limiting feature

31. The anti-reverse fixing structure according to claim 28, wherein a proximal end of the first anti-reverse base defines a second annular step extending toward the mounting substructure, along the axis of the anti-reverse fixing structure, an outer diameter of a proximal end of the second annular step greater than an outer diameter of its distal outer diameter, wherein the mounting substructure comprises a mounting member, the mounting member disposed over an outer side of the distal end of the second annular step, and an inner side of the mounting member being complementary in shape to the outer side of the distal end of the second annular step.

32. The anti-reverse fixing structure according to claim 31, wherein the mounting member is in the shape of a ring, at least one third limiting feature being formed on an inner side of the mounting member, and wherein an outer side of the distal end of the second annular step defines a fourth limiting feature complementary in shape to the third limiting feature.

33. The anti-reverse fixing structure according to claim 24, wherein the anti-reverse substructure comprises a third anti-reverse element and a second support, the third anti-reverse element comprising the first anti-reverse features and a second anti-reverse base, the first anti-reverse features circumferentially arranged on the second anti-reverse base at intervals, a distal end of the second anti-reverse base connected to the proximal end of the fixation helix, a proximal end of the second anti-reverse base connected to a distal end of the second support, and a proximal end of the second support connected to a distal end of the mounting substructure.

34. The anti-reverse fixing structure according to claim 33, wherein the distal end of the second anti-reverse base defines a third annular step extending away from the mounting substructure, along the axis of the anti-reverse fixing structure, an outer diameter of the distal end of the third annular step smaller than an outer diameter of the second anti-reverse base, wherein the first anti-reverse features are disposed on a circumferential side wall of the second anti-reverse base, and wherein the proximal end of the fixation helix is connected to the distal end face of the second anti-reverse base and is disposed over the third annular step.

35. The anti-reverse fixing structure according to claim 34, wherein a proximal end face of the second anti-reverse base is provided with a third locating feature, the third locating feature extending toward the fixation substructure, and wherein the proximal end of the fixation helix is brought into abutment with one side of the third locating feature and causes the fixation helix to be connected to the second anti-reverse base according to a predetermined helical orientation.

36. The anti-reverse fixing structure according to claim 35, wherein the proximal end face of the second anti-reverse base defines a fourth welding surface, the fourth welding surface contiguous with the third locating feature, the fixation helix defining a first welding surface for being welded to the fourth welding surface, and
wherein the first anti-reverse features are located on an outer side of the fourth welding surface.

37. The anti-reverse fixing structure according to claim 36, wherein a projection of the fixation helix along its axis onto the fourth welding surface is encompassed within the fourth welding surface.

38. The anti-reverse fixing structure according to claim 34, wherein the second support comprises a third anti-reverse base, the third anti-reverse base having at least one of the following features:
a distal end of the third anti-reverse base defines a fourth annular step extending toward the fixation substructure, along the axis of the anti-reverse fixing structure, an outer diameter of a distal end of the fourth annular step greater than an outer diameter of its proximal outer diameter, the second anti-reverse base disposed over an outer side of a proximal end portion of the fourth annular step; and
a proximal end of the third anti-reverse base defining a fifth annular step extending toward the mounting substructure, along the axis of the anti-reverse fixing structure, an outer diameter of a distal end of the fifth annular step smaller than an outer diameter of its proximal outer diameter, the mounting substructure comprising a mounting member disposed over an outer side of the distal end of the fifth annular step, and an inner side of the mounting member being complementary in shape to the outer side of the distal end of the fifth annular step.

39. The anti-reverse fixing structure according to claim 38, wherein the third annular step delimits a circular inner bore extending through the second anti-reverse base, and at least one fifth limiting feature is provided on an inner side of the third annular step, the outer side of the proximal end of the fourth annular step defines a sixth limiting feature complementary in shape to the fifth limiting feature, and/or
wherein the outer side of the distal end of the fifth annular step defines at least one seventh limiting feature, the mounting member shaped like a ring, at least one third limiting feature is formed on an inner side of the mounting member, and the seventh limiting feature complementary in shape to the third limiting feature.

40. The anti-reverse fixing structure according to claim 38, wherein between the fourth and fifth annular steps is disposed a support base, and the support base having an outer diameter matching the outer diameter of the second anti-reverse base.

41. The anti-reverse fixing structure according to claim 38, wherein the second support comprises at least one fourth anti-reverse feature, and the at least one fourth anti-reverse feature disposed on a circumference of a distal end face of the fourth annular step and extending in a direction opposite to an unscrewing direction for the fixation helix.

42. The anti-reverse fixing structure according to claim 41, wherein the fixation helix is disposed in a gap between the first anti-reverse features and the fourth anti-reverse feature, and the axis of the fixation helix being oriented parallel to the axis of the anti-reverse fixing structure.

43. The anti-reverse fixing structure according to claim 41, wherein the fourth anti-reverse feature defines a fifth slanted surface and a sixth slanted surface contiguous with the fifth slanted surface, the sixth slanted surface slanted in a direction opposite to the unscrewing direction for the fixation helix, the fifth slanted surface forming a fourth angle with a cross-section of the anti-reverse fixing structure, the sixth slanted surface forming a fifth angle with the cross-section of the anti-reverse fixing structure; wherein both the fourth and fifth angles are non-zero, and the fourth angle is smaller than the fifth angle.

44. The anti-reverse fixing structure according to any one of claims 1 to 43, wherein the mounting substructure further comprises a sealing disc with a through hole, the sealing disc connected to a proximal end of the mounting substructure, and the through hole configured for passage of a feedthrough wire of the implantable medical device therethrough.

45. An implantable medical device, comprising a body of the implantable medical device, an electrode, a feedthrough and the anti-reverse fixing structure of any one of claims 1 to 44,
the electrode connected to a distal end of the anti-reverse fixing structure, a proximal end of the anti-reverse fixing structure connected to a distal end of the body of the implantable medical device, the feedthrough comprising at least one feedthrough wire, which is passed through the anti-reverse fixing structure and electrically connected to the electrode.

46. The implantable medical device according to claim 45, further comprising a drug pill disposed between the electrode and the anti-reverse fixing structure.
